# EUROPEAN PATENT APPLICATION

(11) **EP 4 813 430 A2**
(43) Date of publication of application: **30.09.2026**
(21) Application number: 26184059.9
(22) Date of filing: 16.06.2021
(51) Int. Cl.: A61M 16/00

(54) **IMPROVEMENTS RELATING TO RESPIRATORY SUPPORT**

(30) Priority: 08.07.2020 US 202063049509 P
(62) Document number(s) of the earlier application(s) in accordance with Art. 76 EPC: 21838958.3 / 4 178 424
(71) Applicant: Fisher & Paykel Healthcare Limited, East Tamaki, Auckland 2013 (NZ)
(72) Inventor: TATKOV, Stanislav, 2013 Auckland (NZ)
(74) Representative: Forresters IP LLP

(57) **Abstract**

Described is a method of (and apparatus for) assessing a patient receiving respiratory support during a session to determine a respiratory status comprising: receiving from one or more sensors, for a plurality of time points, one or more patient parameters for a patient, comprising at least one respiratory parameter, determining in a controller: for each time point, a respiratory index from the one or more patient parameters, and a change in respiratory index over time, and determining, from the change in respiratory index over time, a patient respiratory status.

## Description

### FIELD OF THE INVENTION

The present disclosure relates to apparatus, systems and/or methods for using a respiratory index to determine aspects of respiration, including but not limited to respiratory index respiratory status and/or changes in respiratory support (e.g. based on respiratory index and/or status).

### BACKGROUND TO INVENTION

High flow respiratory support has become popular and is commonly used. It has become a front-line therapy for patients with respiratory distress. High flow respiratory support is also used to assist patients whose respiratory systems are compromised (including patients with conditions such as COPD, pulmonary fibrosis, asthma etc).

High flow respiratory support can be an oxygenation tool for patients with respiratory distress or failure. Further, it can increase the amount of O2 delivered due to the fact that high flows can prevent the entrainment of room air. However, high O2 fraction (high FiO2) can potentially mask deterioration of the patient and delay escalation of care.

Patients can be escalated to e.g. mechanical ventilation or non-invasive ventilation. The risk of invasive mechanical ventilation is well understood, although delaying ventilation can result in a lengthened hospital stay and increased mortality.

Therefore, it is desirable to determine when to escalate respiratory support. It can further be desirable to determine a patient's respiratory status to enable a clinician to make better decisions for patients.

### SUMMARY OF INVENTION

In one aspect the present disclosure may be said to comprise a method of assessing a patient receiving NHF respiratory support, and where necessary changing the respiratory support based on the assessment, comprising: receiving from one or more sensors over one or more patient parameters for a patient, comprising at least an oxygenation parameter, determining in a controller a respiratory index at a plurality of times, and determining from a trend in respiratory index over time whether a change in respiratory support is required, and if so, making a change in respiratory support.

In another aspect the present disclosure may be said to comprise a method of treating a patient with a respiratory support apparatus comprising receiving from one or more sensors over one or more patient parameters for a patient, comprising at least an oxygenation parameter, determining in a controller a respiratory index at a plurality of times, and determining from a trend in respiratory index over time whether a change in respiratory support is required, and if so, making a change in respiratory support.

Optionally the respiratory index is ROX index.

Optionally the respiratory index is determined from one or more lung mechanics parameters and one or more oxygenation parameters.

Optionally a lung mechanics parameter is a parameter that indicates lung mechanics, such as respiratory rate, expiratory time, minute ventilation).

Optionally a oxygenation parameter/oxygenation exchange parameter is a parameter that indicates oxygenation, such as SpO2, FiO2, FdO2, O2 fraction... While different, FiO2, FdO2 and O2 fraction can be approximate proxy measurements for each other and can be used interchangeably where appropriate.

Optionally the assessment phase can comprise one or more of:
- assessing the respiratory status, and determining whether it is normal, abnormal, deteriorating, stable, improving or the like,
- assessing whether a change in respiratory support is required (as a result of assessing the respiratory status),
- if the changes required, assessing what change in respiratory support is required (e.g. escalation, de-escalation, increasing or decreasing high flow therapy, escalating to NIV or invasive ventilation, deescalating from NIV or invasive ventilation or the like)

Optionally the change in respiratory support phase can comprise:
- indicating any of the above outcomes of the assessment phase, e.g. by alerts, alarms, messages or other indicators, and/or
- making any of the changes determined in the assessment phase.

Optionally the assessment phase could be implemented:
- by a clinician alone,
- one or more assessment, therapy and/or other apparatus without a clinician,
- or both the clinician and one or more apparatus.

Optionally the respiratory support phase could be implemented:
- by a clinician alone,
- one or more assessment, therapy and/or other apparatus without a clinician,
- or both the clinician and one or more apparatus.

In another aspect the present disclosure may be said to comprise a method of assessing a patient receiving respiratory support during a session to determine a respiratory status comprising: receiving from one or more sensors, for a plurality of time points, one or more patient parameters for a patient, comprising at least one respiratory parameter, determining in a controller: for each time point, a respiratory index and/or one or more component parameters, from the one or more patient parameters, and a change in respiratory index and/or one or more component parameters over time, and determining, from the change in respiratory index and/or one or more component parameters over time, a patient respiratory status.

Optionally the patient is receiving respiratory support, and optionally the respiratory support is: high flow respiratory support, non-invasive pressure respiratory support.

In one aspect, the present disclosure may be said to comprise a method according to any preceding claim wherein a clinician determines a patient respiratory status as "at risk but improving" if: ROX index is below a threshold but the ROX index change indicator showing a trend towards lower risk.

Optionally, if a clinician determines a patient respiratory status as "at risk but improving" then an assessment apparatus provides an indication, such as an initial alarm and display message that indicates the patient is at risk but improving.

Optionally a clinician determines a patient respiratory status as "at risk and deteriorating" if: ROX index is below a threshold and the ROX index change indicator is showing a trend toward higher risk.

Optionally if a clinician determines a patient respiratory status as "at risk and deteriorating" then an assessment apparatus provides an indication, such as an alarm and display message that indicates the patient is at risk and deteriorating.

Optionally a clinician determines a patient respiratory status as "not at risk but deteriorating" if:
ROX index is above a threshold but the ROX index change indicator is showing a trend toward higher risk.

Optionally if a clinician determines a patient respiratory status as "not at risk but deteriorating" then an assessment apparatus provides an indication, such as an alarm quietly, and then alarms loudly if/when the ROX index drops below the threshold.

Optionally:
a clinician determines a patient respiratory status as "stable" if:
respiratory rate is trending upwards (by more than threshold slope or other change indicator) but SpO2 is stable, and
a message is displayed on screen.

Optionally:
a clinician determines a patient respiratory status as "deteriorating" if:
respiratory rate is trending upwards (by more than threshold slope or other change indicator) and SpO2 is trending downwards, and
an alarm is activated.

Optionally, a clinician determines a patient respiratory status from ROX index compared to one or more thresholds.

Optionally, a clinician determines a patient respiratory status from:
respiratory rate,
SpO2, and/or
FiO2
based on one or more thresholds.

Optionally, a clinician determines a patient respiratory status from a change over time of:
respiratory index, and/or
patient parameter, such as respiratory rate, SpO2, and/or FiO2 .

Optionally, a clinician determines a patient respiratory status from a change indicator such as slope, magnitude and/or angle between respiratory index at a plurality of time points.

Optionally, a clinician determines a patient respiratory status from a change indicator such as slope, magnitude and/or angle between a patient parameter, such as respiratory rate, SpO2, and/or FiO2, at a plurality of time points.

Optionally, a clinician determines a patient respiratory status from the length of time a respiratory index and/or change indicator takes to change and/or the magnitude of the change over a threshold time.

Optionally, a clinician determines a patient respiratory status from the time taken to for a respiratory index and/or change indicator by a threshold amount.

Optionally the assessment apparatus and/or respiratory support apparatus have a user interface, such as a display.

In another aspect the present disclosure may be said to comprise a method of assessing a patient receiving respiratory support during a session to determine a respiratory status comprising: receiving from one or more sensors, for a plurality of time points, one or more patient parameters for a patient, comprising at least one respiratory parameter, determining in a controller: for each time point, a respiratory index from the one or more patient parameters, and a change in respiratory index over time, and determining, from the change in respiratory index over time, a patient respiratory status.

Optionally the patient is receiving respiratory support, and optionally the respiratory support is:
high flow respiratory support,

Optionally the session:
is a treatment session,
a day or part thereof,
a night or part thereof,
sub-sessions,
a length of time.

Optionally the one or more patient parameters are one or more lung mechanics parameters and one or more oxygenation parameters.

Optionally a lung mechanics parameter can be one or more of:
- Respiratory rate
- expiratory time,
- minute ventilation.

Optionally a oxygenation parameter can be one or more of:
- FiO2
- FdO2
- O2 fraction
- SpO2

Optionally the respiratory index is ROX index.

Optionally components of the ROX index are: respiratory rate,
SpO2, and/or

FiO2, FdO2, and/or O2 fraction.

Optionally a respiratory rate is determined by the controller from one or more patient parameters received from the one or more sensors.

Optionally further comprising indicating and/or making a change in respiratory support based on the respiratory status and/or respiratory index.

Optionally displaying ROX index numerically and/or graphically.

Optionally determining a change in respiratory index over time comprises:
for a plurality of time points, determining a change in respiratory index over time for each of the plurality of time points.

Optionally further comprising displaying, for the plurality of time points, the change in respiratory index over time for each of the plurality of time points.

Optionally determining a patient respiratory status from the change in respiratory index over time comprises monitoring a change, over the plurality of points in time, of the change in respiratory index over time.

Optionally monitoring a change, over the plurality of time points, of the change in respiratory index over time comprises, for the plurality of time points: viewing the displayed change, over the plurality of points in time, of the change in respiratory index over time ,and/or calculating and comparing the change to relationship information
Optionally further comprising displaying a respiratory index threshold and/or change indicator threshold.

Optionally determining a change in respiratory index over time comprises determining a trend in the respiratory index.

Optionally the trend comprises a plurality of instantaneous trends, and determining a trend comprises determining a plurality of instantaneous trends over time.

Optionally a trend or an instantaneous trend is represented with a trend parameter comprising magnitude and a direction, and optionally could be in the form of: a vector, or a slope and magnitude.

Optionally further comprising communicating the determined change in respiratory support to: a clinician, for example in the form of a message, alarm, respiratory status, respiratory index, and/or a respiratory support apparatus.

Optionally the one or more sensors comprise: one or more sensors arranged to sense a flow path of a respiratory support apparatus, and/or one or more sensors arranged to sense parameters of a patient, and the controller receives the one or more patient parameters from the one or more sensors.

Optionally further comprising displaying on an interface, either on a respiratory apparatus, mobile device and/or other assessment apparatus one or more of: the respiratory index versus time, either graphically and/or numerically, one or more components of the respiratory index (e.g. respiratory rate, SpO2, FiO2 or the like), alone, combined and/or options versus time, either graphically and/or numerically, and/or one or more vectors, slopes, angles, magnitudes, differences and/or other change indicators indicating change between two or more respiratory indexes and/or components thereof, over time or otherwise.

Optionally further comprising receiving input (e.g. user input) to revise the display and re-displaying information based on the user input, comprising one or more of: receiving input to display one or more components of the respiratory index, and displaying the one or more components of the respiratory index alone, combined and/or options versus time, either graphically and/or numerically, and/or receiving input to display, zoom and/or move the display, and displaying, or redisplaying a zoomed and/or moved version of: respiratory index (e.g. ROX index) versus time, either graphically and/or numerically, one or more components of the respiratory index, alone, combined and/or options versus time, either graphically and/or numerically, and/or one or more vectors, slopes, angles, magnitudes, differences and/or other change indicators indicating change between two or more respiratory indexes and/or components thereof, over time or otherwise.

Optionally a clinician determines a patient respiratory status from a change in respiratory index over time by viewing the respiratory index (e.g. ROX index) versus time, either graphically and/or numerically, one or more components of the respiratory index (e.g. respiratory rate, SpO2, FiO2 or the like), alone, combined and/or options versus time, either graphically and/or numerically, and/or one or more vectors, slopes, angles, magnitudes, differences and/or other change indicators indicating change between two or more respiratory indexes and/or components thereof, over time or otherwise.

Optionally a clinician determines a patient respiratory status from a change in respiratory index over time by any one or a combination of the following: comparing one or more respiratory index(es) and/or a change in respiratory index, relative to one or more threshold(s), comparing one or more change indicators relative to one or more threshold(s), comparing one or more respiratory index(es) and/or a change in respiratory index, relative to one or more other respiratory index(es) and/or a change in respiratory index and/or relative to one or more other one or more change indicators, comparing one or more change indicators relative to one or more other change indicators and/or one or more respiratory index(es) and/or a change in respiratory index. considering one or more: respiratory indexes, change in respiratory indexes over time, change in, change in respiratory indexes over time, and/or change indicators.

Optionally upon determining a patient respiratory status, one or more of the following can occur to indicate respiratory status: an alarm is sounded and/or a message is displayed, which indicate the respiratory status, alert the clinician and/or indicate action required, and/or a change in therapy is actioned, automatically and/or manually.

In one aspect, the present disclosure may be said to comprise an apparatus for assessing a patient receiving respiratory support during a session to determine a respiratory status comprising: one or more sensors, or inputs for one or more sensors for receiving from for a plurality of time points, one or more patient parameters for a patient, comprising at least one respiratory parameter, a controller for determining: for each time point, a respiratory index from the one or more patient parameters, and determining, from the change in respiratory index over time, a patient respiratory status, and/or displaying on a display a change in respiratory index over time for a user to determine a patient respiratory status.

Optionally the patient is receiving respiratory support, and optionally the respiratory support is: high flow respiratory support.

Optionally: the assessment apparatus provides the respiratory support, or the assessment apparatus is separate to a respiratory support apparatus.

Optionally the session:
is a treatment session,
a day or part thereof,
a night or part thereof,
sub-sessions,
a length of time.

Optionally the one or more patient parameters are one or more lung mechanics parameters and one or more oxygenation parameters.

Optionally a lung mechanics parameter can be one or more of:
- Respiratory rate
- expiratory time,
- minute ventilation

Optionally an oxygenation parameter can be one or more of:
- FiO2
- FdO2
- O2 fraction
- SpO2

Optionally the respiratory index is ROX index.

Optionally components of the ROX index are: respiratory rate,
SpO2, and/or
FiO2, FdO2, and/or O2 fraction.

Optionally a respiratory rate is determined by the controller from one or more patient parameters received from the one or more sensors.

Optionally the respiratory index is ROX index, determined from respiratory rate, FiO2 and/or SpO2 .

Optionally comprising displaying ROX index numerically and/or graphically on the display.

Optionally determining a change in respiratory index over time comprises:
for a plurality of time points, determining a change in respiratory index over time for each of the plurality of time points.

Optionally comprising displaying, for the plurality of time points, the change in respiratory index over time for each of the plurality of time points.

Optionally determining from the change in respiratory index over time, comprises the controller calculating and comparing the change to relationship information.

Optionally further comprising the controller displaying:
a respiratory index threshold and/or change indicator threshold.

Optionally further comprising communicating the determined change in respiratory support to: a clinician, for example in the form of a message, alarm, respiratory status, respiratory index, and/or a respiratory support apparatus.

Optionally the one or more sensors comprise: one or more sensors arranged to sense a flow path of a respiratory support apparatus, and/or one or more sensors arranged to sense parameters of a patient, and the controller receives the one or more patient parameters from the one or more sensors.

Optionally the apparatus is one or more of a:
respiratory apparatus,
mobile device,
server,
either alone or integrated.

Optionally comprising the sensors.

In another aspect the present disclosure may be said to comprise system for assessing a patient receiving respiratory support during a session to determine a respiratory status comprising:
An apparatus according to any statement herein carrying out a method according to any statement herein

Optionally the apparatus is configured to determine a patient respiratory status from the change in respiratory index over time by monitoring a change, over the plurality of points in time, of the change in respiratory index over time.

Optionally the at least one patient parameter is patient FiO2.

Optionally the respiratory parameters are:
respiratory rate, and/or
SpO2.

Optionally the apparatus is configured to determine a change in respiratory index over time comprises determining a trend in the respiratory index.

Optionally trend comprises a plurality of instantaneous trends, and determining a trend comprises determining a plurality of instantaneous trends over time.

Optionally the change indicator could be in the form of:
a vector, or
a slope and magnitude.

Optionally the apparatus is further configured to display on an interface, either on a respiratory apparatus, mobile device and/or other assessment apparatus one or more of: the respiratory index versus time, either graphically and/or numerically, one or more components of the respiratory index (e.g. respiratory rate, SpO2, FiO2 or the like), alone, combined and/or options versus time, either graphically and/or numerically, and/or one or more vectors, slopes, angles, magnitudes, differences and/or other change indicators indicating change between two or more respiratory indexes and/or components thereof, over time or otherwise.

Optionally the apparatus is further configured to receive input (e.g. user input) to revise the display and re-displaying information based on the user input, comprising one or more of:
receive input to display one or more components of the respiratory index, and displaying the one or more components of the respiratory index alone, combined and/or options versus time, either graphically and/or numerically, and/or
receive input to display, zoom and/or move the display, and displaying, or redisplaying a zoomed and/or moved version of:
   respiratory index (e.g. ROX index) versus time, either graphically and/or numerically.
   one or more components of the respiratory index, alone, combined and/or options versus time, either graphically and/or numerically, and/or
   one or more vectors, slopes, angles, magnitudes, differences and/or other change indicators indicating change between two or more respiratory indexes and/or components thereof, over time or otherwise.

Optionally the apparatus is further configured to allows a clinician to determine a patient respiratory status from a change in respiratory index over time by viewing
the respiratory index (e.g. ROX index) versus time, either graphically and/or numerically,
one or more components of the respiratory index (e.g. respiratory rate, SpO2, FiO2 or the like), alone, combined and/or options versus time, either graphically and/or numerically, and/or
one or more vectors, slopes, angles, magnitudes, differences and/or other change indicators indicating change between two or more respiratory indexes and/or components thereof, over time or otherwise.

Optionally the apparatus is further configured to allow a clinician to determine a patient respiratory status from a change in respiratory index over time by any one or a combination of the following:
compare one or more respiratory index(es) and/or a change in respiratory index, relative to one or more threshold(s),
compare one or more change indicators relative to one or more threshold(s),
compare one or more respiratory index(es) and/or a change in respiratory index, relative to one or more other respiratory index(es) and/or a change in respiratory index and/or relative to one or more other one or more change indicators.
compare one or more change indicators relative to one or more other change indicators and/or one or more respiratory index(es) and/or a change in respiratory index.
consider one or more:
   respiratory indexes,
   change in respiratory indexes over time,
   change in, change in respiratory indexes over time, and/or
   change indicators.

Optionally the apparatus is further configured to upon determining a patient respiratory status, provide one or more of the following can occur to indicate respiratory status:
an alarm is sounded and/or a message is displayed, which indicate the respiratory status, alert the clinician and/or indicate action required, and/or
a change in therapy is actioned, automatically and/or manually.

Optionally a method or apparatus as described wherein the respiratory index is ROX index.

Optionally a method or apparatus as described wherein components of the respiratory index are respiratory rate, SpO2, and/or FiO2
In another aspect, the present disclosure may be said to comprise a method of assessing a patient receiving respiratory support during a session to determine a respiratory status comprising: receiving from one or more sensors, for a plurality of time points, one or more patient parameters for a patient, comprising at least one respiratory parameter, determining in a controller for each time point, a respiratory index and/or one or more component parameters, from the one or more patient parameters, and a change in respiratory index and/or one or more component parameters over time, and determining, from the change in respiratory index and/or one or more component parameters over time, a patient respiratory status.

In another aspect, the present disclosure may be said to comprise a method of assessing a patient to determine a change in respiratory support comprising: Receiving, for a plurality of time points, one or more patient parameters from a patient, comprising at least one respiratory parameter, determining, for each time point, a respiratory index from the one or more patient parameters, from the respiratory index, determining a patient respiratory status, and/or a change in respiratory support based on the trend of the respiratory index.

In another aspect, the present disclosure may be said to comprise a method of assessing a patient to determine a change in respiratory support comprising: receiving, for a plurality of time points, one or more patient parameters from a patient, comprising at least one respiratory parameter, determining, for each time point, a respiratory index from the one or more patient parameters, determining a change in respiratory index over time, from the change in respiratory index, determining a patient respiratory status, and/or a change in respiratory support.

Optionally wherein determining a change in respiratory index over time comprises determining a trend in the respiratory index.

Optionally the trend comprises a plurality of instantaneous trends, and determining a trend comprises determining a plurality of instantaneous trends over time.

Optionally wherein a trend or an instantaneous trend is represented with a trend parameter comprising a magnitude and a direction, and optionally could be in the form of:
a vector, or
a slope (i.e. gradient) and magnitude.

Optionally the method further comprising communicating the determined change in respiratory support to:
a clinician, for example in the form of a message, alarm, respiratory status, respiratory index, and/or
a respiratory support apparatus.

Optionally the method further comprising controlling a respiratory support apparatus based on the determined change in respiratory support.

Optionally the method further comprising determining, from the respiratory index, a patient status and/or change in patient status, and optionally communicating the patient status to a clinician, for example in the form of a message, alarm, and/or status.

Optionally the change in respiratory index, determining a patient respiratory status, and/or a change in respiratory support based on the trend of the respiratory index comprises, comparing one or more of:
- A respiratory index or change in respiratory index
- Trend or plurality of instantaneous trends
- Trend parameter or plurality of trend parameters
- Patient respiratory status or change in respiratory status

against relationship information,
wherein optionally the relationship information comprises:
   at least one threshold, and/or
   a time over which the threshold is met, exceeded or not exceeded.

Optionally the method further comprising communicating one or more of:
- A respiratory index or change in respiratory index
- Trend or plurality of instantaneous trends
- Trend parameter or plurality of trend parameters
- Patient respiratory status or change in respiratory status
- relationship information.

Optionally the respiratory index is ROX index, and the trend parameter is a vector that indicates the change in ROX index and the relationship information is a threshold that indicates risk of respiratory failure.

Optionally the change in respiratory support is an escalation or de-escalation of respiratory support.

Optionally escalating respiratory support comprises:
- providing high flow respiratory support at a higher level. Optionally by increasing or providing flow, O2 concentration, humidification, flow oscillation and/or other high flow parameters
- transferring the patient to a more invasive respiratory support such as:
   ∘ NIV pressure respiratory support
   ∘ Mechanical ventilator respiratory support via intubation

Optionally escalation comprises:
Controlling an apparatus to escalate respiratory support, and/or
Communicating, optionally in the form of message, status, alarm, to a clinician to escalate, or consider escalating, respiratory support.

Optionally the change in respiratory support improves the patient's respiratory status and/or respiratory index.

In another aspect the present disclosure may be said to comprise a method of assessing a patient to determine a change in respiratory support comprising: receiving, for a plurality of time points, one or more patient parameters from a patient, comprising at least one respiratory parameter, determining, for each time point, a respiratory index from the one or more patient parameters, determining at least one vector with magnitude and direction indicating a change of respiratory index over time, determining a change in respiratory support based on the vector.

In another aspect the present disclosure may be said to comprise an apparatus to determine a change in respiratory support comprising: a controller, the controller configured to: receive, for a plurality of time points, one or more patient parameters from a patient, comprising at least one respiratory parameter, determine, for each time point, a respiratory index from the one or more patient parameters, determine a change in respiratory index over time, from the change in respiratory index, determine a patient respiratory status, and/or a change in respiratory support, and an I/O interface to communicate one or more of the: respiratory index and/or change in respiratory index, patient respiratory status, change in respiratory support.

Optionally the apparatus is a respiratory apparatus, comprising a flow generator and a humidifier.

Optionally the flow generator and humidifier are integrated in a housing.

Optionally the apparatus further comprising or configured to couple to one or more of:
a sensor to determine O2 concentration of a gas,
a sensor for determining respiratory rate of a patient.

Optionally the apparatus further comprising a wireless communications transceiver.

Optionally the apparatus is a mobile device with an IO interface and receives patient parameters using one or more of:
Mobile telecommunications
Bluetooth^{™}
NFC.

Optionally the mobile device communicates the:
respiratory index and/or change in respiratory index,
patient respiratory status, and/or
change in respiratory support
to a respiratory apparatus for control of the respiratory apparatus and/or communication on a IO interface of the respiratory apparatus.

In another aspect the present disclosure may be said to comprise a method of controlling a respiratory apparatus comprising: determining a change in respiratory index over time from patient parameters, from the change in respiratory index, determining a patient respiratory status, and/or a change in respiratory support, and communicating to a clinician how to change respiratory support, and/or controlling to a respiratory support apparatus to change respiratory support.

Optionally the change in respiratory support improves the patient's respiratory status and/or respiratory index.

In another aspect the present disclosure may be said to comprise a method of determining one or more trend parameters for a respiratory index: receiving, for a plurality of time points, one or more patient parameters from a patient, comprising at least one respiratory parameter, determining, for each time point, a respiratory index from the one or more patient parameters, determining one or more trend parameters representing a change in the respiratory index over time.

Optionally the trend parameter comprises magnitude and a direction, and optionally could be in the form of:
a vector, or
a slope (i.e. gradient) and magnitude.

In another aspect the present disclosure may be said to comprise a system to determine a change in respiratory support comprising: a mobile device, with a controller, IO interface and a wireless communications transceiver, and a respiratory apparatus, with a controller, flow generator and a humidifier, wherein one or both of the controllers are configured to do some or all of: receive, for a plurality of time points, one or more patient parameters from a patient, comprising at least one respiratory parameter, determine, for each time point, a respiratory index from the one or more patient parameters, determine a change in respiratory index over time, from the change in respiratory index, determine a patient respiratory status, and/or a change in respiratory support.

In another aspect the present disclosure may be said to comprise in an apparatus to determine a change in respiratory support comprising: a mobile device, with a controller, IO interface and a wireless communications transceiver, to receive, for a plurality of time points, one or more patient parameters from a patient, comprising at least one respiratory parameter, determine, for each time point, a respiratory index from the one or more patient parameters, determine a change in respiratory index over time, from the change in respiratory index, convey information on the IO interface, and/or determine a patient respiratory status, and/or a change or suggested change in respiratory support.

Optionally the mobile device receives patient parameters via the wireless communications transceiver using one or more of:
Mobile telecommunications
Bluetooth^{™}
NFC,
WiFi.

Optionally the mobile device receives patient parameters via a WAN, LAN or wireless network.

Optionally the mobile device communicates the:
respiratory index and/or change in respiratory index,
patient respiratory status, and/or
change in respiratory support
to the respiratory apparatus for control of the respiratory apparatus and/or communication on an IO interface of the respiratory apparatus.

Optionally the mobile device and/or respiratory apparatus conveys one or more of the following in a graph, message, display, information, and/or audibly or otherwise in the IO interface:
- A respiratory index or change in respiratory index
- Trend or plurality of instantaneous trends
- Trend parameter or plurality of trend parameters
- Patient respiratory status or change in respiratory status
- Relationship information.

In another aspect the present disclosure may be said to comprise a mobile device and/or a mobile device programmed to carry out a method comprising:
receiving, for a plurality of time points, one or more patient parameters from a patient, comprising at least one respiratory parameter,
determining, for each time point, a respiratory index from the one or more patient parameters,
determining a change in respiratory index over time,
from the change in respiratory index, determining
   a patient respiratory status, and/or
   a change in respiratory support.

In another aspect the present disclosure may be said to comprise a method implemented by a mobile device and/or a mobile device programmed to carry out a method further comprising:
conveying one or more of the following in a graph, message, display, information, and/or audibly or otherwise in an IO interface:
- A respiratory index or change in respiratory index
- Trend or plurality of instantaneous trends
- Trend parameter (e.g. vector, including magnitude and/or direction) or plurality of trend parameters
- Patient respiratory status or change in respiratory status
- Relationship information.

Optionally the system or method may be configured to determine a change in flow rate provided by the respiratory support apparatus required to improve the respiratory index, and may be configured to present instructions on the mobile device to change the flow rate or another parameter of the respiratory support apparatus, optionally wherein the change in flow rate or another parameter is one or more of:
the flow rate is increased to improve the respiratory index
the flow rate is changed based on or relative to the change in the respiratory index
the flow rate is changed based on or relative to the change in trend or trend parameter
FiO2 is be changed relative to respiratory rate change or relative to change in respiratory index.
a gas valve is be controlled to either increase FiO2 or maintain FiO2 while flow rate is changed relative to the index change.

Optionally a system or method wherein the respiratory index is ROX index, which is based on SpO2, FiO2 and respiratory rate, and optionally the system comprises or is configured to connect to one or more sensors from which SpO2, FiO2 and/or respiratory rate can be determined, and optionally wherein respiratory rate is calculated in the controller based on frequency response of an respiratory rate sensor.

Optionally a mobile device captures/receives respiratory rate and FiO2 using an NFC protocol.

Optionally further comprising one or more of:
a respiratory rate sensor
an oxygen concentration sensor
flow sensor (optionally inline)
pressure sensor
temperature sensor
ultrasonic sensor.
and optionally wherein the controller receives signals from one or more of the sensors and/or from manual input and calculates respiratory rate and FiO2 based on received signals.

Optionally a system or method according to any preceding claim wherein: the controller is configured to calculate or the method comprises calculating a trend of a respiratory index over a set period of time based on respiratory rate and FiO2 measurements taken within the set time period, and/or the controller is configured to increase flow from a base flow rate if the trend (or change) in respiratory index indicates respiratory status deterioration, and/or the controller is configured to reduce flow towards a base flow rate if the respiratory index indicates respiratory status improvement.

In another aspect the present disclosure may be said to comprise a method of providing respiratory support comprising: determining a respiratory index of patient respiration at one or more time points, determining a change in respiratory index over time, from the change in respiratory index, determining a patient respiratory status, and/or a suitable respiratory support, and providing the determined respiratory support to the patient.

In one implementation, the embodiment comprises a mobile device receiving information from wearable sensors. The information is used as above and the information conveyed to the clinician and also a respiratory support device to control the device, e.g. through wired or wires transmission, including NFC. The mobile device may communicate with a respiratory support device using Bluetooth or Infra-red or another suitable wireless communication protocol. The mobile device may receive information from the respiratory device and sensors within the respiratory device. The mobile device may automatically ping (i.e. interrogate) the respiratory device at regular time intervals to receive data from sensors onboard the respiratory support device. Alternatively, the respiratory support device may periodically transmit data to the mobile device. In one example NFC communication is advantageous because a user of the mobile device e.g. a clinician can initiate when sensor data from the respiratory support device is received at the mobile device. The mobile device may determine effectiveness of respiratory support using a method as described herein.

In one aspect, the present disclosure may comprise an apparatus for providing respiratory support comprising: a housing, a flow generator (e.g. a blower) within the housing,
a supplementary gases inlet, a valve in fluid communication with the supplementary gases inlet and configured to control the amount of supplementary gases introduced into the apparatus, an outlet located within or on the housing, a gases path extending from the gases inlet to the outlet, through the housing, wherein the flow generator is configured to receive supplementary gases from the supplementary gases inlet and generate a flow of gases, the flow of gases travelling through the gases path, a plurality of sensors, a controller in electronic communication with the one or more sensors and receive signals from the sensors, wherein the sensors are non-invasive sensors, the controller configured to: determine a lung mechanics parameter and an oxygenation parameter from the sensor signals, determine a respiratory index based on the lung mechanics parameter and oxygenation parameter, determine a change in the respiratory index over time, change respiratory support based on the change in the respiratory index over time.

The apparatus optionally comprises a humidifier. The humidifier is positioned downstream of the flow generator, and the humidifier is configured to humidify gases flow.

Optionally, change in respiratory index comprises a trend or a rate of change or a second derivative of the rate of change.

Optionally, the respiratory apparatus may comprise a communication interface that is configured to transmit information to a mobile device (e.g. smartphone or tablet) associated with a clinician or healthcare professional and/or transmit information to a remote patient monitoring system. The remote patient monitoring system may comprise one or more servers, memory units, databases and other components that allow management of patient information, generation of reports of patient's health status and allow alerts to be sent to the patient and/or clinician. The change in respiratory index may be transmitted to the mobile device and/or to the remote patient monitoring system.

The respiratory index measurements and change in respiratory index may be incorporated into a patient report that includes measured patient parameters e.g. SpO2, flow rate, humidity set point and usage hours and the change in respiratory index and respiratory index measured values over time. The

The change in respiratory index allows a clinician to assess if the current therapy being provided is being effective and also allows a clinician to make a change in the therapy provided. In one example the operational parameters of the respiratory support apparatus (e.g. prescription settings) may be remotely updated based on the change in the respiratory index.

In another aspect the present disclosure may be said to comprise a monitoring system comprising:
respiratory support apparatus, (such as a high flow respiratory support apparatus, e.g. a nasal high flow respiratory support apparatus),
a remote monitoring apparatus for a clinician to monitor a patient being supported by the respiratory apparatus,
and one or more controllers in the respiratory apparatus, remote monitoring apparatus and/or other apparatus in the system configured to:
   receive from one or more sensors, for a plurality of time points, one or more patient parameters for a patient, comprising at least one respiratory parameter,
   determine in the one or more controllers,
      for each time point, a respiratory index from the one or more patient parameters, and
      a change in respiratory index over time,
         and
provide information (numerically, graphically or otherwise) to the remote monitoring apparatus, the information being one or more of:
   respiratory index, and/or
   change in respiratory index over time.

Optionally one or more of the following could also be provided
- patient respiratory status,
   change in respiratory status over time,
- patient parameter versus time
- change patient parameter versus time
- respiratory index threshold,
- change indicator threshold,
- suggestions of respiratory support.

It is intended that reference to a range of numbers disclosed herein (for example, 1 to 10) also incorporates reference to all rational numbers within that range (for example, 1, 1.1, 2, 3, 3.9, 4, 5, 6, 6.5, 7, 8, 9 and 10) and also any range of rational numbers within that range (for example, 2 to 8, 1.5 to 5.5 and 3.1 to 4.7) and, therefore, all sub-ranges of all ranges expressly disclosed herein are hereby expressly disclosed. These are only examples of what is specifically intended and all possible combinations of numerical values between the lowest value and the highest value enumerated are to be considered to be expressly stated in this application in a similar manner.

The term "comprising" as used in this specification means "consisting at least in part of". When interpreting each statement in this specification that includes the term "comprising", features other than that or those prefaced by the term may also be present. Related terms such as "comprise" and "comprises" are to be interpreted in the same manner. Unless the context clearly requires otherwise, throughout the description and the claims, the words "comprise", "comprising", and the like, are to be construed in an inclusive sense as opposed to an exclusive or exhaustive sense, that is to say, in the sense of "including, but not limited to".

In this specification where reference has been made to patent specifications, other external documents, or other sources of information, this is generally for the purpose of providing a context for discussing the features of the disclosure. Unless specifically stated otherwise, reference to such external documents is not to be construed as an admission that such documents, or such sources of information, in any jurisdiction, are prior art, or form part of the common general knowledge in the art.

The disclosure may also be said broadly to comprise in the parts, elements and features referred to or indicated in the specification of the application, individually or collectively, in any or all combinations of two or more of said parts, elements or features. Where, in the foregoing description reference has been made to integers or components having known equivalents thereof, those integers are herein incorporated as if individually set forth.

To those skilled in the art to which the disclosure relates, many changes in construction and widely differing embodiments and applications of the disclosure will suggest themselves without departing from the scope of the disclosure as defined in the appended claims. The disclosures and the descriptions herein are purely illustrative and are not intended to be in any sense limiting. Where specific integers are mentioned herein which have known equivalents in the art to which this disclosure relates, such known equivalents are deemed to be incorporated herein as if individually set forth. The disclosure comprises the foregoing and also envisages constructions of which the following gives examples only.

### BRIEF DESCRIPTION OF DRAWINGS

Embodiments of the will now be described with reference to the following drawings, of which:
Figure 1 shows a flow diagram of an assessment phase and respiratory support phase of the present disclosure for determining respiratory support requirements of a patient.
Figure 2 shows a graph of a respiratory index versus time with respect to a threshold relating to patient respiratory status.
Figure 3 shows a system for implementing the assessment phase and respiratory support phase.
Figure 4 shows a graph of ROX index versus time with respect to a threshold relating to patient respiratory status.
Figure 5 shows a graph of Respiratory rate versus FiO2 with vectors of ROX index over time with respect to a threshold relating to patient respiratory status.
Figure 6 shows a respiratory support apparatus.
Figure 7 shows a mobile device and screen displaying information for assessing patient respiration.
Figures 8 to 10 show use cases of the method and apparatus described.
Figures 11A to 11E show displayed information for example use cases.

### DETAILED DESCRIPTION OF EMBODIMENTS

### Terms

Breathing assistance apparatus, respiratory apparatus, respiratory support apparatus, breathing apparatus can all be interchangeably used to define the same apparatus.

**Respiratory index** - an indicator of a patient's respiration, for example, and an indicator of respiration and/or gases exchange in the patient. Respiratory index is a parameter from which respiratory status and/or decisions about respiratory support provided to the patient can be determined. For example, a respiratory index might indicate increasing severity of respiratory distress to allow a clinician to escalate therapy to a more severe therapy (e.g. NIV or intubation). A respiratory index could be determined from/is a function of: one or more lung mechanics parameters (such as respiratory rate, expiratory time, minute ventilation), and one or more oxygenation parameters (such as SpO2, FiO2, FdO2, O2 fraction,...).

In an alternative characterisation, the respiratory index may be considered a unitless figure that is characterised as a function f(x) of one or more:
- Patient parameters, which might comprise among other things:
   ∘ physiological parameters, (which can comprise respiratory parameters), and
   ∘ therapy parameters (therapy delivered to the patient);
      and
- respiratory apparatus parameters (which can comprise operational parameters).

**Lung mechanics parameter** - this is a parameter that indicates lung mechanics, such as respiratory rate, expiratory time, minute ventilation).

**Oxygenation parameter/oxygenation exchange parameter** - this a parameter that indicates oxygenation, such as SpO2, FiO2, FdO2, O2 fraction. While different, FiO2, FdO2 and O2 fraction can be approximate proxy measurements for each other and can be used interchangeably where appropriate.

**O2 fraction -** is the fraction of oxygen in a gases stream.

**FiO2 -** the fraction of inspired oxygen by a patient
**FdO2 -** the fraction of oxygen delivered to a patient
**SpO2 -** is blood oxygen concentration in a patient.

**Respiratory state** - the current state of a patient's respiration. The status could indicate normal respiration, or respiratory distress. It can be an indicator and/or a result of respiration and gases exchange. Respiration status will be affected by lung mechanics (such as respiratory rate) and gases exchange (that is, blood gases exchange - such as indicated by FiO2 requirements). Respiratory status can change over time

**Respiratory status** - a indicator of a patient's current and/or possible future respiratory state. It comprises the respiratory state, but also any past or future changes or trends in the state that indicate the overall wellbeing of patient both now and possible well-being in the future. This can be used to predict the likely course of patient well-being and decide what action, if any, is needed.

**Respiratory distress** - when a patient's respiration is not normal. For example, respiratory distress might be e.g. hypoxemic respiratory distress, acute respiratory distress syndrome, hypercapnic respiratory distress, dyspnoea, or respiratory compromise. Respiratory distress can be on a scale from mild to severe (e.g. respiratory failure). Respiratory distress might, for example, range from mild to severe and may present itself as one or more of:
patient difficulty breathing,
patient has increased respiratory rate,
deterioration of breathing towards respiratory failure,
onset of respiratory failure,
occurrence of respiratory failure,
an increase in O2 requirement e.g. increased FiO2 to maintain a level of SpO2, abnormality of patient blood gases level,
Dyspnoea,
Low SpO2,
High PaCO2,
high likelihood of decompensation.

Respiratory distress happens first. Respiratory failure may follow on after respiratory distress.

**Respiratory failure** - develops when a patient's lungs cannot get enough oxygen into their blood, and may manifest as an abnormality in patient blood gases and/or abnormality in breathing. The degree of respiratory failure can be indicated by respiratory rate and level of blood oxygen. The sicker the patient the more O2 required and/or higher the respiratory rate. For example, respiratory failure can manifest as and/or be indicated by an increase in respiratory rate beyond resting respiratory rate - e.g. double the resting respiratory rate.

**Risk of respiratory failure** - indicates the risk of onset of respiratory failure

**Change indicator** - indicates a change in respiratory index (or other parameter) over time (or over any other parameter change). It could be a slope, vector, angle, magnitude, difference, or the like - be it numerical or graphical. A reference to any particular change indicator, e.g. slope, is generally used by way of example only and it will be appreciated that other change indicators could convey the same or similar information and generally a reference to a particular change indicator can be considered to be interchangeable with another change indicator.

**High flow respiratory support** - In general terms, this provides a high flow of gas to support respiration of a patient. For example, this can be supplied by a nasal cannula in nasal high flow respiratory support (nasal high flow respiratory support (NHF)) , or by a trachea interface (e.g. tracheostomy adapter) in tracheal high flow respiratory support. the term "high flow respiratory support" can be taken to mean one or more, without limitation, of the following terms and types of respiratory support used by those skilled in the art. Note, some of these are the similar terms used for the same type of respiratory support:
- high flow
- high flow oxygen
- humidified high flow
- high flow nasal oxygen
- nasal high flow
- tracheal high flow
- high flow delivery
- high flow therapy
- humidified high flow nasal cannula

High flow respiratory support can be useful for respiratory distress and respiratory failure.

**Non-invasive (NIV) pressure respiratory support** - This is ventilatory support for a patient. It controls ventilation by providing Bi-Level pressure therapy. This therapy is a non-invasive pressure therapy. For example, Bi-Level pressure therapy, where higher pressure is provided on inspiration and lower pressure on expiration. This allows control of tidal volume and PEEP at least. NIV is ventilatory support and controls ventilation. NIV is administered with a sealed interface. The terms NIV, NIV pressure respiratory support and Bi-level pressure support can be used interchangeably.

**Invasive respiratory support** - in general terms this is mechanical ventilation provided to an intubated patient.

**Base respiratory support** - this is the initial respiratory support provided by a clinician, typically via nasal high flow or tracheal high flow respiratory support.

**High flow** - (for example with respect to high flow respiratory support) means, without limitation, any gas flow with a flow rate that is higher than usual/normal, such as higher than the normal inspiration flow rate of a healthy patient. It can be provided by a non-sealing respiratory system with substantial leak happening at the entrance of the patient's airways due to non-sealing patient interface, such as a nasal cannula. High flow is provided as part of high flow respiratory support as defined above, such as in nasal high flow or tracheal high flow. It is also provided with humidification to improve patient comfort, compliance, and safety. Alternatively or additionally, it can be higher than some other threshold flow rate that is relevant to the context - for example, where providing a gas flow to a patient at a flow rate to meet or exceed inspiratory demand, that flow rate might be deemed "high flow" as it is higher than a nominal flow rate that might have otherwise been provided. "High flow" is therefore context dependent, and what constitutes "high flow" depends on many factors such as the health state of the patient, type of procedure/therapy/support being provided, the nature of the patient (big, small, adult, child) and the like. Those skilled in the art know from context what constitutes "high flow". It is a magnitude of flow rate that is over and above a flow rate that might otherwise be provided.

But, without limitation, some indicative values of high flow can be as follows.
- In some configurations, delivery of gases to a patient at a flow rate of greater than or equal to about 5 or 10 litres per minute (5 or 10 LPM or L/min).
- In some configurations, delivery of gases to a patient at a flow rate of about 5 or 10 LPM to about 150 LPM, or about 15 LPM to about 95 LPM, or about 20 LPM to about 90 LPM, or about 25 LPM to about 85 LPM, or about 30 LPM to about 80 LPM, or about 35 LPM to about 75 LPM, or about 40 LPM to about 70 LPM, or about 45 LPM to about 65 LPM, or about 50 LPM to about 60 LPM. For example, according to those various embodiments and configurations described herein, a flow rate of gases supplied or provided to an interface via a system or from a flow source, may comprise, but is not limited to, flows of at least about 5, 10, 20, 30, 40, 50, 60, 70, 80, 90, 100, 110, 120, 130, 140, 150 LPM, or more, and useful ranges may be selected to be any of these values (for example, about 20 LPM to about 90 LPM, about 40 LPM to about 70 LPM, about 40 LPM to about 80 LPM, about 50 LPM to about 80 LPM, about 60 LPM to about 80 LPM, about 70 LPM to about 100 LPM, about 70 LPM to about 80 LPM).
- In some configurations typical flow rates for adults often range from, but are not limited to, about fifteen litres per minute (LPM) to about seventy litres per minute or greater. Typical flow rates for paediatric patients (such as neonates, infants, and children) often range from, but are not limited to, about one litre per minute per kilogram of patient weight to about three litres per minute per kilogram of patient weight or greater. High flow can also optionally include gas mixture compositions including supplemental oxygen and/or administration of therapeutic medicaments. The flow rates used to achieve "high flow" may be any of the flow rates listed below. For example, in some configurations, for an adult patient 'high flow respiratory support' may refer to the delivery of gases to a patient at a flow rate of greater than or equal to about 10 litres per minute (10 LPM), such as between about 10 LPM and about 100 LPM, or between about 15 LPM and about 95 LPM, or between about 20 LPM and about 90 LPM, or between 25 LPM and 75 LPM, or between about 25 LPM and about 85 LPM, or between about 30 LPM and about 80 LPM, or between about 35 LPM and about 75 LPM, or between about 40 LPM and about 70 LPM, or between about 45 LPM and about 65 LPM, or between about 50 LPM and about 60 LPM. In some configurations, for a neonatal, infant, or child patient 'high flow respiratory support' may refer to the delivery of gases to a patient at a flow rate of greater than 1 LPM, such as between about 1 LPM and about 25 LPM, or between about 2 LPM and about 25 LPM, or between about 2 LPM and about 5 LPM, or between about 5 LPM and about 25 LPM, or between about 5 LPM and about 10 LPM, or between about 10 LPM and about 25 LPM, or between about 10 LPM and about 20 LPM, or between about 10 LPM and 15 LPM, or between about 20 LPM and 25 LPM. A high flow respiratory support apparatus with an adult patient, a neonatal, infant, or child patient, may deliver gases to the patient at a flow rate of between about 1 LPM and about 100 LPM, or at a flow rate in any of the sub-ranges outlined above.
- The flow therapy apparatus 10 can deliver any concentration of oxygen (e.g., FdO2), up to 100%, at any flowrate between about 1 LPM and about 100 LPM. In some configurations, any of the flowrates can be in combination with oxygen concentrations (FdO2s) of about 20%-30%, 21%-30%, 21%-40%, 30%-40%, 40%-50%, 50%-60%, 60%-70%, 70%-80%, 80%-90%, and 90%-100%. In some combinations, the flow rate can be between about 25 LPM and 75 LPM in combination with an oxygen concentration (FdO2) of about 20%-30%, 21%-30%, 21%-40%, 30%-40%, 40%-50%, 50%-60%, 60%-70%, 70%-80%, 80%-90%, and 90%-100%. In some configurations, the flow therapy apparatus 10 may include safety thresholds when operating in manual mode that prevent a user from delivering to much oxygen to the patient.
- Flow rates for "High flow" for premature/infants/paediatrics (with body mass in the range of about 1 to about 30 kg) can be different. The therapeutic flow can be set to 0.4-8 L/min/kg with a minimum of about 0.5 L/min and a maximum of about 25 L/min. For patients under 2 kg maximum flow is set to 8 L/min. The oscillating flow is set to 0.05-2 L/min/kg with a preferred range of 0.1-1 L/min/kg and another preferred range of 0.2-0.8 L/min/kg.

In "high flow" the gas delivered will be chosen depending on for example the intended use of a therapy, of which some examples are above. Gases delivered may comprise a percentage of oxygen. In some configurations, the percentage of oxygen in the gases delivered may be about 15% to about 100%, 20% to about 100%, or about 30% to about 100%, or about 40% to about 100%, or about 50% to about 100%, or about 60% to about 100%, or about 70% to about 100%, or about 80% to about 100%, or about 90% to about 100%, or about 100%, or 100%.

### 1. Overview

Embodiments described herein provide apparatus, systems, and methods for assessing a patient's respiratory status (e.g. normal, distressed, deteriorating, or improving, stable) ("assessment phase" or "diagnostic phase"); and based on that assessment optionally taking appropriate respiratory support action ("respiratory support phase"). For example, if a patient is in respiratory distress, or is progressing to or in respiratory failure in a respiratory support phase, then escalation of respiratory support might be made to reduce further deterioration of respiratory status. The assessment is preferably made when the patient is receiving respiratory support, e.g. high flow respiratory support, NIV, invasive ventilation or the like.

In the assessment phase, a respiratory index can be determined. The respiratory index is an indicator of patient respiration, and the respiratory index (and/or change in respiratory index) can be used to determine a patient's (current) respiratory state and/or change in respiratory state - therefore leading to a determination of respiratory status.

A respiratory status can be "normal", or "respiratory distress". Respiratory distress can range from mild to severe, as will be described later. The respiratory index can indicate if a patient is trending towards or experiencing: onset of respiratory distress or is in respiratory distress, and/or is experiencing or is trending towards deteriorating respiratory distress. (Deteriorating respiratory distress can lead to risk of or actual respiratory failure, which is a severe type of respiratory distress).

From the patient respiration, respiratory index and/or respiratory status, it can be determined if it is desirable to escalate respiratory support to improve the patient's respiratory status/relieve respiratory distress (in a respiratory support phase). This might be to prevent further deterioration of respiratory distress (stabilisation) or to move the patient out of respiratory distress into normal breathing. For example, if the patient is in mild respiratory distress, escalation of respiratory support might be used to reduce the chances that the patient deteriorates to a more severe level of respiratory distress, such as risk of respiratory failure, or actual respiratory failure. Or, if a patient is already at risk of respiratory failure (or has experienced respiratory failure) escalation of respiratory support reduces the risks of respiratory failure occurring (or continuing) and/or the negative health outcomes of respiratory failure.

However, escalating respiratory support (such as increasing high flow respiratory support, providing NIV pressure respiratory support, or providing invasive respiratory support) has its own risk, so it is undesirable to escalate respiratory support unnecessarily. Likewise, when respiratory distress has lessened, it is often desirable to de-escalate respiratory support so as to remove the risk encountered due to the escalated respiratory support. Among other things, a reason for the assessment phase is to identify early enough if a patient's respiratory status is deteriorating so that action can be taken pre-emptively without doing so unnecessarily early. Escalating respiratory support early improves health outcomes, whereas delay can risk negative health outcomes.

For example, a patient might be receiving base respiratory support, in the form of high flow respiratory support e.g. nasal high flow respiratory support or tracheal high flow respiratory support. The assessment phase can be used to determine if escalating respiratory support could benefit the patient and should be implemented.

In one example, escalation of respiratory support can comprise escalating high flow respiratory support. This might take the form of increasing the high flow respiratory support parameters (e.g. flow rate, O2 concentration, humidification, or the like) of respiratory support, while de-escalating respiratory support can comprise reducing the support parameters. Such escalation might occur, for example, when it is determined from the respiratory index that the patient is in respiratory distress and is deteriorating, but is not yet at high risk of respiratory failure. Escalating high flow respiratory support might stabilise or even improve the patient respiratory status, meaning risk of respiratory failure (and hence even more invasive escalation) is avoided.

As another example, escalation of respiratory support can comprise moving to a more invasive respiratory support. This might be an escalation to NIV respiratory support, or invasive respiratory support. This might happen immediately or after escalation of high flow respiratory support. In the case of nasal/tracheal high flow respiratory support, escalation of respiratory support can also comprise going from nasal high flow to invasive ventilation, such as providing mechanical ventilation to an intubated patient. De-escalation can comprise:
if it is being used, removing invasive respiratory support and returning to the base respiratory support (for example, NIV respiratory support or nasal high flow), or
if it is being used, removing NIV respiratory support and returning to the base respiratory support (for example, nasal high flow), or
if it is being used, de-escalating nasal high flow).

Alternatively, as another example of more invasive respiratory support, escalation of respiratory support can comprise transferring the patient to non-invasive ("NIV") pressure respiratory support. This might happen immediately or after escalation of high flow respiratory support. De-escalation can comprise removing NIV pressure respiratory support and returning to the base respiratory support (for example, nasal high flow).

Respiratory support can comprise using humidification also, for example with nasal high flow, tracheal high flow and/or NIV pressure respiratory support. As another example, escalation of respiratory support can comprise providing, or escalating, humidification. This can be for therapy and/or comfort, and might instead of or be in addition to any one of the other escalations described. De-escalation can comprise removing or reducing humidification.

Therefore, the present embodiments utilise the change in respiratory index (such as a trend in respiratory index) to help ascertain when it is appropriate to escalate and/or de-escalate respiratory support. Using the respiratory index trend helps to improve the timing of the escalation of therapy as required to help improve stabilisation of the patient (and improve respiratory index, status etc.).

This provides improved health outcomes (including improved respiratory index, status etc.) as escalated respiratory support is provided when it is more likely that the benefits of providing escalated support outweigh the risk of providing escalated support.

Typically, the embodiments described relate to base respiratory support in the form of nasal and/or tracheal high flow respiratory support, which is high flow of gas provided to a patient to support respiratory function. Preferably, the high flow respiratory support comprises humidification for patient comfort.

In general terms, the present apparatus and methods comprise one or more of:
an assessment method,
carried out by an assessment apparatus, and
optionally a method and/or apparatus for providing respiratory support based on the assessment method outcome.

This allows a clinician and/or apparatus to determine a suitable change in respiratory status/state, e.g. changes in respiratory distress or deterioration toward respiratory failure, and allows a clinician and/or respiratory apparatus to escalate respiratory support sooner. For example, a patient can be intubated and provided with mechanical ventilation earlier than under current diagnostic methods. The methods described herein allow for early detection of deterioration of a patient respiratory state e.g. increasing respiratory distress or deterioration that is indicative of potential respiratory distress (be that mild or severe). This allows a clinician/respiratory apparatus to escalate respiratory support earlier, which can increase chances of recovery and increase chances of survival.

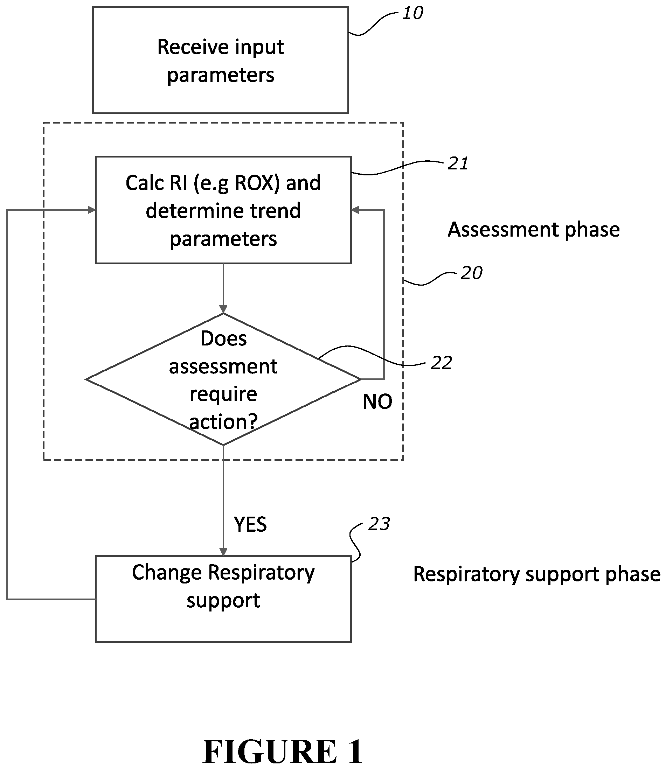

Referring to Figure 1, in general terms, the apparatus and/or methods in combination can:
a) in an assessment phase determine:
   a respiratory index, and
   determining from the change in respiratory index the respiratory status of the patient and/or if respiratory support changes are required,
   and
b) in a respiratory support phase, based on the assessment phase,
   implement (by either the clinician and/or respiratory apparatus) respiratory support changes using a respiratory apparatus.

It is not necessary to have both a) and b). For example, just an assessment stage a) might be implemented.

The assessment phase can comprise one or more of:
- assessing the respiratory status, and determining whether it is normal, abnormal, deteriorating, stable, improving or the like,
- assessing whether a change in respiratory support is required (as a result of assessing the respiratory status),
- if the changes required, assessing what change in respiratory support is required (e.g. escalation, de-escalation, increasing or decreasing high flow therapy, escalating to NIV or invasive ventilation, deescalating from NIV or invasive ventilation or the like)

The implementation and the respiratory support phase can comprise:
- indicating any of the above outcomes of the assessment phase, e.g. by alerts, alarms, messages or other indicators, and/or
- making any of the changes determined in the assessment phase.

The assessment phase could be implemented:
- by a clinician alone,
- one or more assessment, therapy and/or other apparatus without a clinician,
- or both the clinician and one or more apparatus.

Likewise, the respiratory support phase could be implemented:
- by a clinician alone,
- one or more assessment, therapy and/or other apparatus without a clinician,
- or both the clinician and one or more apparatus.

A respiratory index ("RI") is determined, and then optionally an assessment of the patient respiratory status is made from the change in respiratory index. Then, based on either the change in respiratory index and/or the patient respiratory status (which e.g. might be displayed to a clinician), optionally a decision is made whether a change (e.g. escalation or de-escalation) of respiratory support is required, and if so, what change. Optionally a respiratory apparatus is configured to provide the change in support, either automatically or by intervention by a clinician.

A respiratory index could be determined from/is a function of: one or more lung mechanics parameters (such as respiratory rate, expiratory time, minute ventilation), and one or more oxygenation parameters (such as SpO2, FiO2, FdO2, O2 fraction). These parameters may be calculated by a controller of the respiratory apparatus based on signals/measurements from the one or more sensors associated with the respiratory apparatus.

Alternatively the respiratory index could be characterised as follows: it is may be a unitless figure that is a function f(x) of one or more:
- Patient parameters, which might comprise among other things:
   ∘ physiological parameters, (which can comprise respiratory parameters), and
   ∘ therapy parameters (therapy delivered to the patient);
      and
- respiratory apparatus parameters (which can comprise operational parameters).

Physiological parameters can comprise lung mechanics and/or oxygenation parameters.

Typically, a respiratory index may use one or more of SpO2 (patient physiological parameter), FiO2 (patient therapy parameter), and respiratory rate (patient physiological parameter). Note, FdO2 could be used as a proxy for FiO2. FdO2 is the fraction of delivered oxygen, which is the O2 concentration of the gases stream and FiO2 is the fraction of inspired oxygen. They are related and similar in value, but not the same -although if the gas flow is high enough and a patient does not entrain ambient air, FdO2 will be substantially equivalent to FiO2 so FdO2 can be used as a proxy for FiO2. FdO2 is a respiratory apparatus parameter. With respiratory apparatus that provide oxygen, the oxygen concentration (an operational parameter) provided by the apparatus can be closely related to FiO2 and can be used as a proxy of FiO2. But other parameters can be used also. The respiratory index can be a function of any set of parameters that provides an indication of respiration, from which a determination of a patient respiratory status (such as the risk of respiratory failure) can be made.

For example, the respiratory index RI could be defined as in general a function of:
- one parameter representative of lung mechanics and one parameter representative of oxygen exchange, that RR = f(LM, O), or
- respiratory rate (RR) - e.g. RI=f(RR), or,
- a function of respiratory rate and FiO2 - e.g. RI=f(FiO2, RR), or
- a function of respiratory rate, FiO2, and SpO2 - e.g. RI=f (SpO2, FiO2, RR).

In one example, the respiratory index can be ROX which is defined as: $ROX = \left(SpO2/FiO2\right) / RR$ where
SpO2 is the setpoint saturation of oxygen (%) in the blood of a patient, or alternatively the actual saturation of oxygen (%) in the blood of a patient,
FiO2 is the fraction of inspired oxygen (%) by the patient (FdO2 could be used as a proxy), and
Respiratory rate is the breathing rate in breaths per minute.

A change in the respiratory index RI, e.g. over time, may be used to determine patient respiratory status. From the respiratory status optionally a determination can be made about whether the respiratory support needs changing. Or, from the from the change in respiratory index itself, optionally a determination can be made about whether the respiratory support needs changing.

For example, the change in respiratory index itself might provide an indication of whether a change in respiratory support is required. Or, for example, some relationship between the change in respiratory index and some other information ("relationship information"); for example, a parameter (e.g. threshold - predetermined or otherwise) can provide that determination. Multiple values of the respiratory index might be used to make a determination (e.g. multiple values of the respiratory index might be determined over time, and from that a trend determined which can be determined with a respiratory changes required). The relationship between multiple values of the respiratory index, or some information such as a parameter that generalises the multiple values and another parameter could be used. There are various options for determining whether respiratory support needs changing, which are underpinned by the respiratory index but optionally in addition to other information.

Looking at the change in respiratory index over time could comprise looking at multiple time instances of the respiratory index changing over time. For example, for each of a plurality of points in time, a (e.g. instantaneous) change in respiratory index over time (at that point in time) could be determined. This change (over the plurality of points of time) of the (e.g. instantaneous) change in respiratory index (over time) can be used to determine a patient respiratory status. That is, for example, a derivative of the respiratory index over time could be found at multiple times, and those multiple time instances of the derivative of respiratory index over time could also be differentiated over time to give a second derivative of respiratory index over time. For example, this could be an acceleration in the change of: respiratory index, patient parameter, patient state, and/or patient status. The first and/or second derivatives could be viewed or compared to relationship information (e.g. slope threshold other change indicator threshold) to assess patient respiratory status. For example, the derivative of respiratory index over time and/or the second derivative of respiratory index over time could be displayed in numerical and/or graphical form. Monitoring a change, over the plurality of points in time, of the change in respiratory index over time comprises, for the plurality of points in time: viewing the displayed change in respiratory index over time and/or calculating and comparing the change to relationship information. The change in respiratory index over time can be indicated by a change indicator.

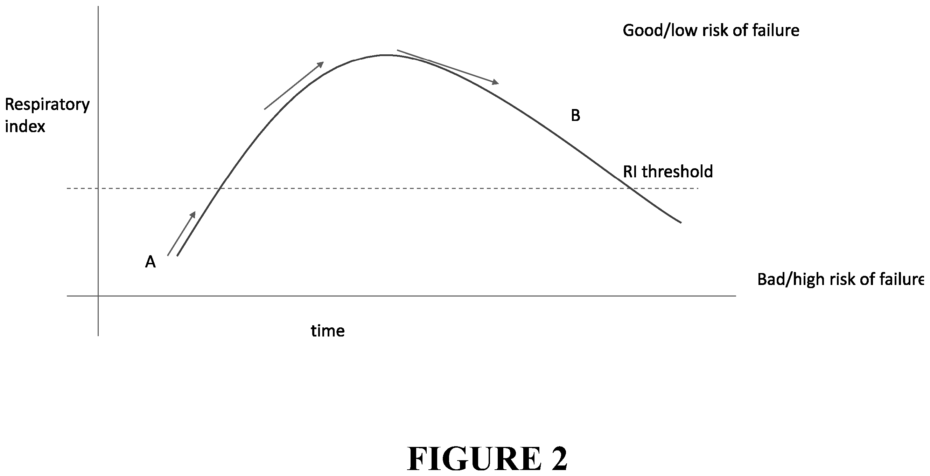

As an example, a desirable option would be to look at a respiratory index over time and determine the trend of whether the respiratory index increases or decreases or changes in some other manner, or otherwise has some relationship change with respect to relationship data, such as a threshold (for example as shown in Figure 2). From any of the above, an indication of respiratory status can be determined and from that respiratory support changes made. Or optionally the respiratory index and its trend (such as its magnitude and direction) relative to a threshold can be used to directly determine if respiratory support changes required. The assessment using the respiratory index can provide a trigger for respiratory support changes.

A threshold or other relationship information might be determined through experimental data, clinical research, user input, calculation, and/or otherwise. The threshold or other relationship might be predetermined or determined in real-time or input or otherwise provided. The threshold or other relationship might be fixed or varying depending on other parameters (for example it may change over time, or may change depending on other parameters used in the respiratory index or alternatively not used in the respiratory index).

Non-limiting examples of a threshold could be a respiratory index threshold, which delineates normal v distressed respiratory status, and/or a slope threshold (or other "change indicator" threshold, depending on the indicator being used that indicates a change in respiratory index), which might delineate between a trend toward improvement and deterioration of respiratory index. There could be one or more respiratory index or slope thresholds.

As some examples, the respiratory index and/or change in respiratory index can be assessed using one or a combination of the following:
- Comparing the respiratory index (e.g. ROX) v one or more respiratory index threshold(s).
- Comparing the change in respiratory index over time v one or more respiratory index threshold(s)
- Comparing a change indicator (e.g. vector, slope or other measure of magnitude and/or direction) of change in respiratory index over time v a threshold (e.g. one or more slope threshold(s))
- Comparing respiratory index v patient parameter(s), or some function of patient parameters(s) (such as SpO2, FiO2 or the like), such as in graphical and/or numerical form that can be displayed and monitored
- Comparing the change in magnitude (drop or climb) of the respiratory index over time to a threshold(s). e.g. a slow (that is, small slope) but significant difference (that is large delta in ROX) in respiratory index at time=1 v respiratory index at time =2 might indicate deterioration. Likewise, a large but slow positive change in magnitude could be an indication of improvement.

Comparing the change of any of the above versus time as a first derivative to some relationship data, and/or comparing that change in time versus time (acceleration of change) as a second derivative to some relationship
The examples above refer to the use of a respiratory index to determine the respiratory status of a patient.

In an alternative, it is possible to use respiratory index and patient parameters (which could be constituent components of the respiratory index) to make the assessment. Therefore, in more general terms, one or more respiratory parameter(s) can be used to assess the patient status along with a respiratory index. This can be particularly useful when a clinician is making the assessment and wants to drill down deeper into what is causing the respiratory status. The respiratory index itself can indicate the respiratory status (e.g. a change in respiratory state) but may not indicate why that change is happening. In contrast, patient parameters such as respiratory rate, FiO2 and/or SpO2 might provide insights into that. This will be described in further detail later. Respiratory index, patient parameters and any other information which relates to patient state or status to make an assessment can be termed "assessment information". Assessment criteria can be used to help with the assessment.

The above is not limiting. The respiratory status assessment can be more generally carried out by:
displaying assessment information, assessment criteria and/or relationship information as numbers, plots and/or or other graphical indications on a display (either static or animated and/or in 2 or 3 dimensions - for example with time on one axis), and a person viewing that information, and/or
an apparatus comparing the above to relationship information.

The optional determination of whether respiratory support changes are required based on using the respiratory index can be termed a "diagnostic stage" or alternatively a "trigger". Alternatively, the assessment can be considered the diagnostic stage, irrespective of whether a course of action is determined.

In many cases, it is the clinician that will assess the respiratory status based on information displayed on the assessment apparatus. By displaying various combinations of graphical and numerical representations of parameters and respiratory indexes, it is possible for a trained clinician to interpret these to gain an indication of respiratory status, and in particular the direction of respiratory state and what interventions might be required. This enables a clinician to make decisions quickly in situations where there might be many patients that are being monitored simultaneously - e.g. in an Emergency Department. These assessments might be assisted by indications (alarms, messages and the like) from the assessment apparatus and/or automated decisions made by an assessment apparatus. Similarly this same advantage can be achieved for homecare patients that is, a clinician monitoring multiple patients remotely can quickly assess a patient that is deteriorating or potentially going to suffer respiratory failure.

Traditionally, a clinician has had to use subjective measures based on limited information to assess a patient's respiratory status. Additionally they have had to use invasive testing methods. The present embodiments improve on this. In general terms, the assessment can be made by displaying graphically and/or numerically a respiratory index, a change indicator, patient parameters and/or thresholds etc. in various combinations to provide the supporting information for a clinician to make the decision. Numerical information, two or three-dimensional plots, animations, moving plots, zooming in and drilling down into information and contextual information can also be used to make an assessment. Providing visual, contextual and/or accessible information will assist a clinician to make quick decisions in an objective manner, as opposed to having to use solely subjective considerations. This also enables treatment by exception - addressing those who need help most first. Examples will be described herein later.

The assessments (determination) are carried out during a session. A session might be defined by a treatment session, a day or part thereof, a night or part thereof, sub-sessions, or a length of time (e.g. 5 hours) for example. Generally, the comparisons are made relative to thresholds and/or previous respiratory index parameters determined in the same session. That is, the assessments are based on what is happening in real-time. Assessments might be made on a time-period-by-time-period basis, where each time period is a part of a session. E.g. assessments might be made on a minute-by-minute basis, ten minute-by-ten minute basis, hour-by-hour basis or the like. Over this time frame, assessment can comprise considering the change between respiratory index and/or constituent parameters from time period to time period. Such assessments can be based on the change in respiratory index and/or constituent parameters either alone and/or compared to relationship information also. Assessment (determination) can also alternatively be made within and/or across multiple sessions. For example, over multiple sessions, assessment can comprise considering the change between respiratory index and/or constituent parameters from session to session (and/or within a session). Such assessments can be based on the change in respiratory index and/or constituent parameters either alone and/or compared to relationship information also.

In a home environment, the clinician will most likely not be situated with the patient. In that case, patient respiratory index/status can be monitored remotely. For example this could be by way of an assessment apparatus that provides assessment information remotely to a clinician and/or might be by way of the clinician possessing the assessment apparatus, which remotely communicates with the respiratory support apparatus and/or any other apparatus (such as sensors) that are required to make the assessment. The respiratory support apparatus and/or assessment apparatus will have communications functionality 55B, 55A to enable the clinician to monitor the patient remotely. For example, a remote assessment apparatus may calculate the respiratory index and/or changes in the respiratory index based on measurements from the respiratory support apparatus as with the patient. The respiratory index may be calculated in the respiratory index values provided to the clinician. The processing may take place on the respiratory apparatus and the processed information provided to the clinician, and/or alternatively, the clinician as an assessment apparatus that receives the raw data and makes the assessment. There might be a remote patient monitoring system 57, e.g. comprising at least a remote server 57, which receives information from the assessment apparatus and/or respiratory support apparatus, via a network 56 which the clinician can access. For example, the clinician might access the information via a web browser/web server. The assessment apparatus might be the server, respiratory apparatus, mobile device and/or any other assessment apparatus.

The remote patient monitoring system 57 further comprises one or more databases, a reporting engine to generate patient reports and other suitable components that allow monitoring of a patient, generation of patient reports (e.g. that include usage of a respiratory support apparatus, therapy settings etc.). The remote patient monitoring system 57 allows a clinician to manage multiple patients remotely e.g. allow management of patient's while the patients are at home (i.e. outside hospital).

The respiratory index data may be provided to the remote patient monitoring system 57. The respiratory index measurements and change in respiratory index may be incorporated into a patient report that includes measured patient parameters e.g. SpO2, flow rate, humidity set point and usage hours and the change in respiratory index and respiratory index measured values over time.

The change in respiratory index allows a clinician to assess if the current therapy being provided is being effective and also allows a clinician to make a change in the therapy provided. In one example the operational parameters of the respiratory support apparatus (e.g. prescription settings) may be remotely updated based on the change in the respiratory index. The change in the respiratory index can be used by the remote monitoring system to generate alerts or messages to clinicians and patients. The remote patient monitoring system 57 may automatically change therapy settings e.g. change high flow therapy settings such as oxygen fraction (i.e. % oxygen in gases) and/or flow rate and/or humidity (e.g. dew point or relative humidity or absolute humidity) remotely, based on the changes in the respiratory index. Examples of how therapy settings (i.e. therapy parameters) are changed are described later. The therapy settings, specifically for example high flow respiratory support apparatus settings may be changed and transmitted as a new prescription to the respiratory support apparatus 10 via the network 56. The respiratory support apparatus 10 may incorporate these changes and begin to operate based on the new prescription (i.e. updated therapy settings i.e. updated therapy parameters). Alternatively a clinician or healthcare professional may make a change to the therapy settings at the remote monitoring system 57 based on the changes in the respiratory index. These clinician changes are transmitted to the respiratory support apparatus 10 via the network 56. The new settings are transmitted as a new prescription. The respiratory support apparatus makes changes defined in the new prescription (i.e. updates its therapy settings) to operate as per the new settings.

The process of monitoring a respiratory index can continue. This remote monitoring provides an effective out of hospital monitoring of the patient. It allows clinicians to assess patient respiratory status and determine if high flow therapy is assisting the patient by checking the change in the respiratory index. The change in respiratory index is indicative of a change in the respiratory status of the patient. The disclosure provides an effective out of hospital monitoring of the patient. It further allows remotely changing therapy settings a respiratory support apparatus associated with a patient based on the assessed change in respiratory index.

Once a determination has been made (trigger), the respiratory support changes are determined. The support the respiratory support changes could be determined by a clinician and/or by the assessment apparatus.

This might result in one or more changes to use of the respiratory support apparatus, be that:
operational changes (either automatic or clinician instigated) such as increasing flow or oxygen fraction provided to a patient;
use changes (such as intubating a patient instead of providing high flow through an unsealed cannula), including providing entirely different therapy - e.g. ventilation,
provision of indications (e.g. alerts, alarms and/or instructions) to a clinician that:
   a change needs to be made,
how and/or when to change therapy, and/or
configurational changes.

These are not limiting of the possible respiratory support change options.

As part of the assessment phase, irrespective of whether it is carried out by a person and/or an apparatus, an indication and/or change in therapy may be used to indicate that there has been a determination made and/or there has been a change in therapy and/or there should be a change in therapy and/or what a change in therapy should be. For example:
▪ Audible alarm - this may be made to indicate a determination has been made, what the determination is (e.g. worsening) and/or that some therapy change will or may have occurred. The alarm may be made on the therapy device or a separate device (e.g. a doctor's phone). Different sounds may indicate different patient states (or different changes to a patient's state).
▪ Visual alarm - this may be made to indicate a determination has been made, what the determination is (e.g. worsening) and/or that some therapy change will or may have occurred. The alarm may be on the therapy device or a separate device. Different visual outputs may indicate different patient states (or different changes to a patient's state).
▪ Message - this may be made to indicate a determination has been made, what the determination is (e.g. worsening) and/or that some therapy change will or may have occurred. The message may be sent to one or more recipients/devices (each recipient/device may receive a different message or the same message). This message may include a warning and/or suggest a change to one or more device set points. The message may include a suggestion for the specific value(s) that the set point(s) should be changed to. The calculation of these specific values could be performed on the therapy device or on a separate device.
▪ Automatic change to device set point(s) - after a determination (e.g. worsening patient status) a controller associated with the device may change one or more set points if the index value/slope suggests that the patient needs different support, for example, more flow. The calculation of the suitable change(s) could be performed on the therapy device or on a separate device.
▪ Device turns off - after a determination (e.g. improving patient status) a controller associated with the device may turn the therapy/device off if the index value/slope suggests that the patient no longer needs the therapy (i.e. if their index value/slope is on the low-risk side of a threshold and/or is moving in a good direction).

The above could be provided on the apparatus itself or remotely to another apparatus, where a clinician might be based remotely.

This list is not exhaustive.

The assessment apparatus might be in the vicinity or possession of the clinician. This might be in a hospital environment, for example. Alternatively, the assessment apparatus might be remote from the clinician. It would have communications functionality to enable communication to a remote clinician. This might be in a home environment, for example, where the clinician is not situated with the patient/respiratory apparatus and/or assessment apparatus. In another alternative, the respiratory apparatus might be with the patient, but the assessment apparatus remote with a remote clinician elsewhere.

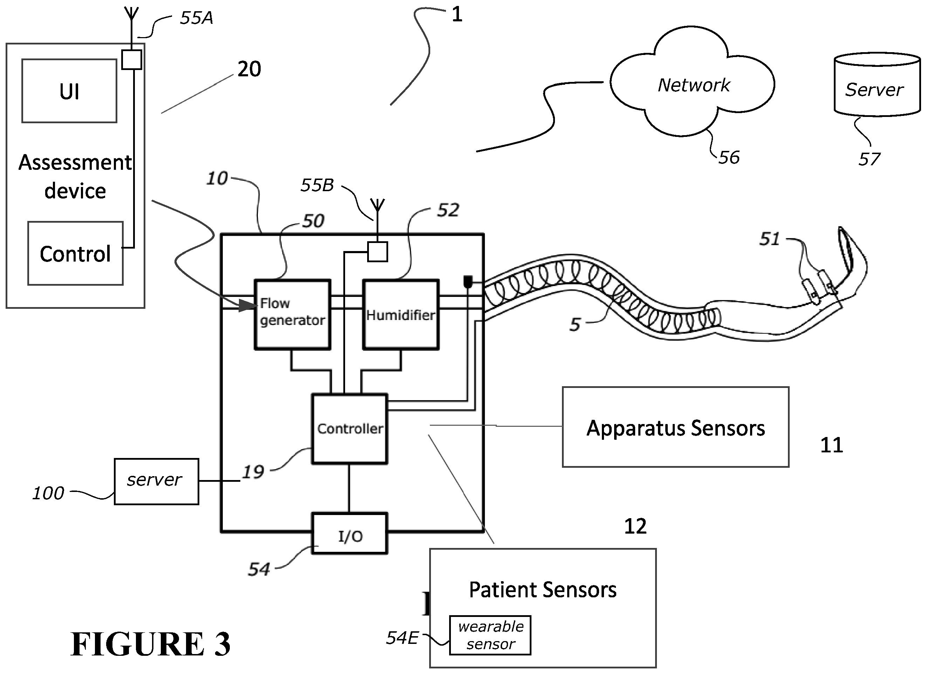

As noted above, the embodiments described will typically be related to use of (initially at least) a nasal or tracheal high flow respiratory support apparatus 10, generally shown in Figure 3. But this is not limiting, and for example, the embodiments could be used in relation to NIV or other respiratory apparatus. There is the apparatus 10 which provides the respiratory support and the apparatus 20 which determines the diagnostics. These may be one and the same apparatus (e.g. respiratory support apparatus) or different apparatus (e.g. respiratory support apparatus and a mobile device). If separate apparatus (as in Figure 3), together they can form a respiratory assessment and support system 1.

For example, referring to Figure 3, there could be a respiratory support apparatus that has a controller (could also be referred to as a processor) 19, I/O interface 54, flow generator 50 and humidifier 52. The respiratory support apparatus could be configured to receive air and/or supplementary gas such as oxygen. It could provide a flow of gas to a patient through a breathing conduit 5 and patient interface 51, e.g. an unsealed interface (such as but not limited to a nasal cannula) or tracheal interface. The controller 19 can operate the device to provide the required flow rate and/or pressure, temperature, humidity, oxygen or other gas fraction and the like, based on input from sensors 11 in the system or to which the system is connected. They could be non-invasive sensors.

The apparatus also has (integrated within it) and/or connects to sensors 12 that provide:
- physiological parameters, (which can comprise respiratory parameters), and
- respiratory apparatus parameters (which can comprise operational parameters)
to obtain parameters for operation of the apparatus (such as temperature, humidity, pressure, flow sensors) and parameters to determine the respiratory index (such as SpO2, FiO2 (or FdO2 - fraction of delivered oxygen to a patient), and respiratory rate). Any reference to FiO2 could be replaced with a reference to FdO2, O2 fraction, or any other oxygenation parameter and vice versa - the variations can be used interchangeably. Physiological parameters can comprise lung mechanics and/or oxygenation parameters.

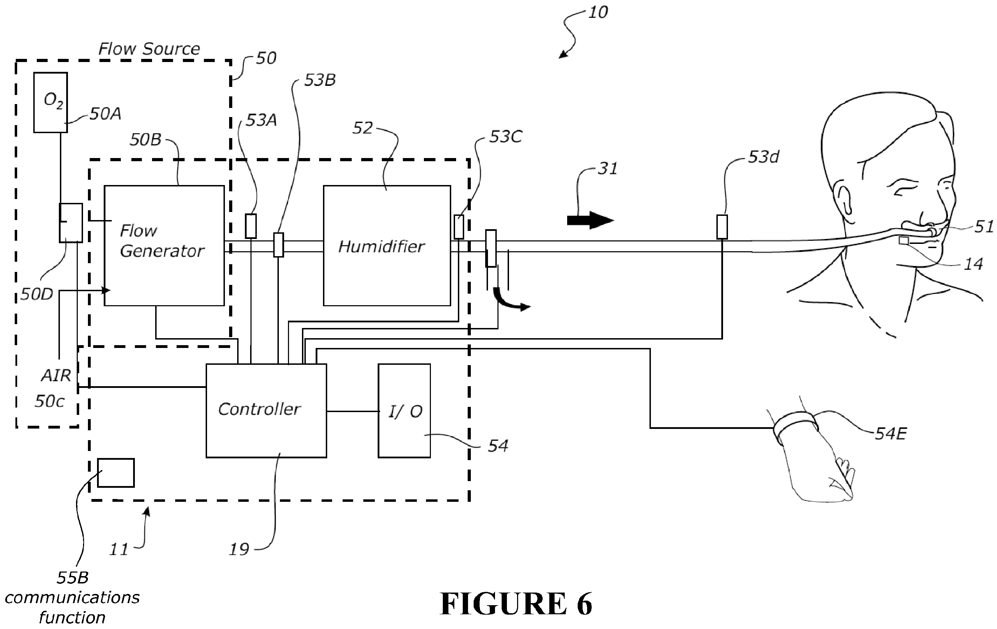

As possible examples:
Note, any of these could be wearable (e.g. see 54E in Figure 6).

For SpO2 the apparatus could connect to physiological sensors such as pulse oximeter or other blood oxygen sensor. This may be a wearable device e.g. see 54E in Figure 6. There could be a wireless pulse oximeter (SpO2 sensor). Wireless pulse oximeter could communicate via Bluetooth or infrared or other communications protocol. Wireless SpO2 allows patient mobility e.g. patient can walk around while still using SpO2 and the assessment apparatus still receives measurements. The SpO2 sensor connects to the respiratory or assessment apparatus. The respiratory apparatus is a non-invasive sensing unit that also provides respiratory support. The respiratory apparatus can process the sensor signals and calculate the index/change in respiratory index.

Alternatively change in respiratory index could be done at a remote monitoring system.

For respiratory rate, this can be measured/determined using a pressure sensor or a flow sensor or any other respiratory rate sensor from which respiratory rate can be determined. For example, respiratory rate can be calculated using a flow sensor, and then applying a mathematical process to the flow sensor readings to determine respiratory rate. In one example the respiratory support apparatus is configured to use the flow sensor, process the flow sensor to filter out the flow signals from the blower. A frequency response of the filtered flow signal is determined (e.g. a Fast Fourier Transform or other frequency response) to calculate a peak frequency. The peak frequency corresponds to the respiratory rate. Other frequency analysis techniques may be applied to the flow signal to determine a respiratory rate. For example, the frequency analysis can determine one or more local maxima, and identify the highest magnitude maxima as the respiratory rate. Alternatively, a motor speed signal is processed (e.g. filtered) from the flow signal and frequency analysis is conducted, where the local maxima and frequency with the highest magnitude is respiratory rate. Alternatively, a frequency analysis using Geortzel algorithm/Geortzel analysis might be done.

Alternatively, respiratory rate measurement can be achieved by having a temperature sensor at the cannula e.g. incorporated into the cannula and determining temperature differential. Difference in temperature is indicative of respiratory rate. A similar measurement can be achieved by pressure sensor or flow sensor at the cannula and changes in pressure or flow can be indicative of respiratory rate.

Alternatively respiratory rate may be determined from other sensors such as (but not limited to), a pulse oximeter, a respiratory rate sensor, a pressure sensor in the device; or a pressure sensor or flow sensor in the tube or patient interface; or chest band sensors or any combination thereof. One or more of these could optionally be a wearable device or an actigraphy device (e.g. see 54E in Figure 6) that is configured to measure respiratory rate. The wearable device may be, e.g., a wrist-worn device that is in wireless communication with the flow generator or smartphone.

For example, the respiratory rate is calculated by based on pressure signals. The pressure signal can be measured by a pressure sensor at the patient interface or using a pressure pathway, the sensor can be in the device and the pressure values can be communicated back to the device. The differences in pressure can be processed and the respiratory rate can be calculated by measuring the number of 0 crossings. A similar approach can be used using flow readings. The cyclic nature of the P or F signal can be processed to get a respiratory rate.

Alternatively, a suitable sensor e.g. a respirometer can be used to measure the gases exchange (e.g. CO2 exchange) at the interface to determine respiratory rate. Alternatively, a photoplethysmogram or an electrocardiogram can be used to measure.

Alternatively, any sensor, such as an accelerometer can be placed on the chest or an abdominal wall to measure respiratory rate. The sensor can be separate from the device with wireless communication.

Alternatively, respiratory rate could be determined as described in US202101133796 which is incorporated herein in its entirety.

In one example expiratory time may be calculated from flow sensor signals or pressure signals or a combination of flow and pressure sensor signals. Below is an example of expiratory time calculation for an unsealed system that provides high flow respiratory support via an unsealed cannula e.g. apparatus 10 disclosed herein. The method is executed by the controller. The controller of the apparatus receives a flow signal representative of a flow rate of the gases. The controller pre-process and filters the flow rate signal. The controller then determines a primary breathing parameter ratio e.g. a ratio between inspiratory time and total respiratory time, and/or expiratory time and total respiratory time for a patient's breathing cycle. Determine or receive respiratory rate. Respiratory rate may be determined as per above or received manually at the respiratory support apparatus. The apparatus uses the breathing parameter ratio and the respiratory rate to determine an expiratory time. Further minute ventilation may be determined from the respiratory rate and the measured flow rate. In a further example expiratory time may be calculated as described in US provisional application US 63/146,184 filed on 5 February 2021, the contents of which are incorporated herein in its entirety.

The measured or calculated respiratory rate is stored in the NHF therapy device. The FiO2 readings are also stored in the NHF device over a period of time. Alternatively, or additionally, these and/or any other readings might be transmitted to and stored in another device, such as a smart phone.

Optionally, respiratory rate may be manually inputted via user interface.

Other options are possible also.

FiO2 can be found by measuring FdO2 or another measure of concentration of O2 in the gas flow using any suitable sensor e.g. an ultrasonic sensor or other gas concentration sensor. For example, this could be done with a flow sensor and O2 concentration sensor, where optionally the flow sensor is an inline flow sensor. This sensor could be positioned downstream of a mixer of ambient air and O2.

An ultrasound sensor could be used to allow fast measurement of FiO2 that is, FdO2. This allows for fast response and control of oxygen in the gases stream. As mentioned earlier FdO2 can be a proxy for FiO2. The measured FdO2 is reported and presented on a screen of the apparatus as FiO2. During high flow respiratory support the flow rate is sufficiently high such that ambient air is not entrained at the nasal cannula when the patient is inspiring. Therefore the delivered oxygen fraction FdO2 is denoted as equivalent to FiO2 (inspired oxygen fraction). The ultrasound (i.e. ultrasonic) sensor allows for fast response i.e. quick measurement and therefore fast response. A valve on the oxygen inlet can be controlled to change the oxygen fraction in the gases stream (thereby affecting FdO2).

Any of the parameters above could be manually input instead and respiratory index calculated from that.

The assessment apparatus and/or respiratory support apparatus might also have respective communications functionality. This could be a modem or other transceiver. This enables the assessment apparatus and the respiratory support apparatus to communicate with each other, whether remote or in the same location, and/or also communicate to a remote clinician. This would enabler clinician to remotely monitor patient and their respiratory support and/or change as required. There might be a remote server, which receives information from the assessment apparatus and/or respiratory support apparatus, which the clinician can access. For example, the clinician might access the information via a web browser/web server. The assessment apparatus might be the server, respiratory apparatus, mobile device and/or any other assessment apparatus

A high flow respiratory apparatus 10 in general is described with reference to e.g. Figure 3. In general terms, the apparatus comprises a main housing 10 that contains a flow generator 50 in the form of a motor/impeller arrangement, an optional humidifier 52, a controller 19, and a user I/O interface (comprising, for example, a display and input device(s) such as button(s), a touch screen, or the like). An input for supplementary oxygen or other supplementary gas can be provided. A valve (e.g. proportional valve) can be provided in fluid communication with the supplementary gases inlet and configured to control the amount of supplementary gases introduced into the apparatus. The screen may be a detachable screen. The controller 19 is configured or programmed to control the components of the apparatus, including: operating the flow generator to create a flow of gas (gas flow) for delivery to a patient, operating the humidifier (if present) to humidify and/or heat the generated gas flow, receive user input from the I/O interface for reconfiguration and/or user-defined operation of the apparatus, and output information (for example on the display) to the user. The user could be a patient, healthcare professional, or anyone else interested in using the apparatus. A patient breathing conduit is coupled to a gas flow output in the housing of the flow therapy apparatus, and is coupled to a patient interface 51 such as a nasal cannula with a manifold and nasal prongs. The patient breathing conduit can have a heater wire 5 to heat gas flow passing through to the patient.

Some examples of high flow respiratory apparatus are disclosed in International Application No. PCT/NZ2016/050193, titled "Flow Path Sensing for Flow Therapy Apparatus", filed on December 2, 2016, and International Application No. PCT/IB2016/053761, titled "Breathing Assistance Apparatus", filed on June 24, 2016, which are hereby incorporated by reference in their entireties. Examples of configurations of high flow respiratory apparatus that can be used with aspects of the present disclosure are also discussed in further detail below.

The respiratory support apparatus might determine the respiratory index and/or change in respiratory index and make the assessment leading to any changes in respiratory support. Alternatively, the relevant information may be communicated to a separate assessment device where the determination of the respiratory index, change in respiratory index, patient respiratory status and/or any changes in respiratory support can be made. Information regarding change in respiratory support can then be communicated back to a clinician and/or the respiratory support apparatus for the appropriate action. Additionally, or alternatively, information might be transferred to a physician server 100 or a remote server. For example, the clinician might access the information via a web browser/web server. The assessment apparatus might be the server, respiratory apparatus, mobile device and/or any other assessment apparatus. There can be wired and/or wireless communication between the respiratory support apparatus and clinician device, such as a smartphone.

The respiratory support apparatus may comprise a controller configured to control the blower to provide Bi-level pressure therapy. The respiratory support apparatus may be coupled to a sealed interface e.g. a full face mask. Optionally an NIV tube may be coupled that has a lower resistance to flow than the high flow tube (as shown in figures). A user may manually select an NIV mode (e.g. Bi-level pressure therapy or CPAP therapy) mode and the controller is configured to operate in the selected mode. Alternatively the respiratory support apparatus could be configured to detect the connection of a sealed interface and/or a connection of an NIV tube and automatically change control.

In a further alternative a patient may be physically coupled to a suitable pressure support device e.g. a NIV device.

In general terms, there are various embodiments of a respiratory index and how a change in respiratory index is used to assess patient respiratory status and/or a change in respiratory support; and any of these embodiments can be used in combination with any of various embodiments of respiratory support apparatus and (where applicable) a separate assessment device to carry out:
the assessment phase, and optionally
the respiratory support phase (information on and/or change in respiratory support)

### 2. Assessment method

### 2.1 Overview of assessment method

Referring to Figure 1, a change in respiratory index is used to determine an appropriate respiratory support action. As an interim step, a patient respiratory status might be determined from the change in respiratory index, but that is not essential.

A respiratory index could be determined from/is a function of: one or more lung mechanics parameters (such as respiratory rate, expiratory time, minute ventilation) and one or more oxygenation parameters (such as SpO2, FiO2, FdO2, O2 fraction).

The respiratory index may be a unitless figure that is a function f(x) of one or more patient parameters being:
- physiological parameters, (which can comprise respiratory parameters), and
- respiratory apparatus parameters (which can comprise operational parameters)

Physiological parameters can comprise lung mechanics and/or oxygenation parameters.

A respiratory index can be calculated based on sensor inputs and/or user inputs that provide the above data. A respiratory index as an example is based on respiratory rate (respiratory rate) and FiO2 (oxygen concentration delivered to patient). The index is preferably related to 1/RR and/or 1/FiO2. In one example the index = A/B (RR*FiO2), where A and B could be constants or other values.

One example of a respiratory index is a ROX index.

A ROX index takes the form of a function $ROX \left(x\right) = f \left(FiO2, SpO2, RR\right)$ and is calculated as follows: $ROX = \left(SpO2/FiO2\right) / RR$

Where:
SpO2 is the setpoint saturation of oxygen (%) in the blood of a patient, or alternatively the actual saturation of oxygen (%) in the blood of a patient,
FiO2 is the fraction of inspired oxygen (%) by the patient (FdO2 could be used as a proxy), and
respiratory rate is the breathing rate in breaths per minute.

The above are "input parameters", received via sensors and/or a user via a I/O interface or preconfigured, step 10.

The SpO2 is received for example from a sensor, such as a pulse oximeter, or from apparatus SpO2 set point. Respiratory rate is, for example, received from a sensor such as described previously. FiO2 can be measured for example via a sensor or inferred from the oxygen concentration (e.g. FdO2) provided by the apparatus to a patient. These are examples only. More details of how the information is obtained is described with respect to the apparatus embodiments.

Where ROX index is used, in a preferred embodiment the ROX index threshold for respiratory failure is 4.88, which has been determined by experimental data. In that case, if the ROX index is greater than or equal to about 4.88 the patient is deemed to be at low risk of respiratory failure. However, if the ROX index is below about 4.88 then the patient is deemed to be at high risk of respiratory failure. It should be noted that a clinically insignificant deviation from 4.88 as the threshold is possible and should not be excluded from scope of the embodiment. Clearly also, the threshold could be a different value, where appropriate.

However, having simply a single instance in time of ROX index relative to the threshold in itself may not be sufficient information to determine whether the current therapy settings are resulting in effective treatment for the patient. The trend over time of the ROX index (or more generally respiratory index) and/or it's relation to the threshold may also be useful.

In an assessment phase step 20, a respiratory index (e.g. ROX index) can be calculated, step 21, and then a change in respiratory index over time can be determined and used to determine success of high flow respiratory support and whether change is required, step 22. For ROX index, if the FiO2 and respiratory rate increase, the ROX index begins to drop in value. An increase in FiO2 and respiratory rate is indicative of the patient deteriorating. The continuous monitoring of the ROX index (resulting in a change of ROX index) is helpful when the patient is an unstable condition.

As an example, referring to Figure 1, the apparatus receives input parameters, step 10, and determines the respiratory index (e.g. ROX index) and the change in respiratory index over time - for example through calculation, step 21. Optionally, an assessment is then made as to the respiratory status of the patient based on the change of respiratory index over time. This can be done by determining the relationship of the change in respiratory index over time to other information. For example, the change in respiratory index over time is compared to a threshold to determine if the patient respiratory status is "respiratory distress" or even further a level of distress such as "severe" (e.g. the patient has a high risk of respiratory failure).

An assessment of a change in respiratory support can optionally be determined, step 22, based on the patient respiratory status (or directly from the ROX index or other respiratory index as it changes over time). (Because the patient respiratory status can be assessed based on the relationship of the respiratory index change over time to a threshold, then an assessment of the change in respiratory support, step 22, 23, can alternatively be considered to be determined based on the relationship of the respiratory index change over time relative to the threshold). Using the ROX index over time and the threshold, the change in respiratory support, step 23, can be an escalation of respiratory support, or a de-escalation of respiratory support. Details of a change in respiratory support (either escalation, de-escalation or otherwise) is described later with respect to the apparatus embodiments.

The example above refers to the use of a respiratory index to determine respiratory status of a patient. As noted earlier, in an alternative, it is possible to use patient parameters (which could be constituent components of the respiratory index) to make the assessment. Therefore, in more general terms, one or more respiratory parameter(s) can be used to assess the patient status instead of and/or as well as a respiratory index.

In many cases, it is the clinician that will assess the respiratory status based on information displayed on the assessment apparatus. Displaying various combinations of graphical and numerical representations of parameters and respiratory indexes makes it possible for a clinician to gain an indication of respiratory status, and in particular the direction of respiratory state and what interventions might be required.

In the case of clinician assessment, the display of information assists the clinician, both in terms of the information displayed in the manner that can be reviewed and accessed.

In general terms, the assessment can be made by displaying graphically and/or numerically respiratory index, a change indicator, patient parameters and/or thresholds etc. in various combinations to provide the supporting information for a clinician to make the decision. Examples will be described herein later.

Any one or more of the following parameters could be displayed numerically and/or graphically in any suitable combination to assist clinician assessment. Any particular parameter in itself might not warrant action, but when considered in combination with other information may indicate that action is required. For example, a ROX index on its own may not flag any concerns, but a worsening change in the ROX index over time might do so. Or, even a worsening change in ROX index over time might not be cause for concern, unless it is also accompanied by a concerning change in some other parameter, such as respiratory rate, SpO2 or FiO2 for example. Also, absolute or relative changes in any of the above might not be any cause for concern, unless they exceed a certain threshold. By providing access to various combinations of such information, a clinician gets a richer set of information from which to assess respiratory status.

Some of the following parameters (assessment information, assessment criteria and/or relationship information) that could be used alone or in combination are:
- respiratory index
- change in respiratory index over time
- patient parameter (e.g. respiratory rate, SpO2, FiO2)
- change in patient parameter over time
- change indicator (such as slope, vector, magnitude, difference, angle) that indicates the change (of any parameter herein) over time or over another measure
- respiratory index threshold
- change indicator threshold.
- These could be displayed graphically (that is as a 2D and/or 3D plot/graph or other suitable visual output) or numerically, for example as some of the following:
- respiratory index versus time
- change in respiratory index this is time
- patient parameter versus time
- change patient parameter versus time
- respiratory index threshold
- change indicator threshold.
- Input can be received from the user to manipulate graphical information to get more insights. For example, this could comprise:
- zooming in and/or moving a graph
- drilling into a graph to get further graphs on underlying parameters, e.g. selecting a respiratory index versus time graph and then getting patient parameter versus time plots.
the above is not limiting, and various examples will be described later.

Possible examples now follow, but should not be considered limiting to the above more general embodiment.

### 2.2 Embodiment 1 - assessment method - respiratory index trend

In this embodiment, referring to Figure 1, a respiratory index is determined over time, and the change in respiratory index over time is used to assess respiratory status and/or changes in respiratory support.

The respiratory index could be any as previously described such as a function of:
- one parameter representative of lung mechanics and one parameter representative of oxygen exchange, or
- respiratory rate - e.g. RI(RR), or,
- a function of respiratory rate and FiO2 - e.g. RI (FiO2, RR), or
- a function of respiratory rate, FiO2 and SpO2 - e.g. RI(SpO2,FiO2, RR)

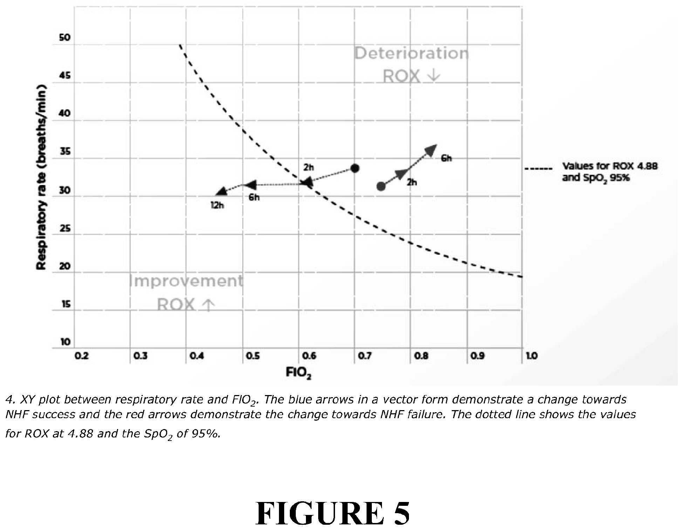

The respiratory index change over time is assessed, step 20. Optionally, the patient's respiratory status is assessed and for example, in effect, a trend in the change in respiratory index is used to indicate patient respiratory status. Referring to Figure 2 or 5, if the respiratory status trends towards a better value (e.g. goes towards, crosses or goes beyond a threshold value) and/or trends towards a better value at or above a threshold rate, then an assessment can be made that the patient respiratory status is good or is improving. In contrast, if the respiratory status trends towards a worse value (e.g. moves away from or crosses or goes beyond a threshold value in the other direction) and/or trends towards a worse value at or above a threshold rate, an assessment can be made that the patient respiratory status is bad or is deteriorating. From this, decisions about any change in respiratory support can be made. It should be noted that respiratory status does not actually need to be determined. Decisions about change in respiratory support, step 22, 23, can be made from the change in respiratory index over time without explicitly determining respiratory status. But the relationship between respiratory index and respiratory status can be used to predetermine suitable respiratory support actions when a particular respiratory index or change in respiratory index (e.g. trend) occurs.

The trend can be characterised in any suitable way, in the form of a respiratory index change (trend) parameter. This characterises the trend/change both in terms of magnitude and direction. As an example, the trend could be in the form of a vector showing the change in respiratory index (over e.g. time but it could be with respect to another suitable parameter). In addition, information about a threshold value which indicates crossing between a lower risk value and a higher risk value for the respiratory index can be utilised to assess the trend. The vector might be found, for example, from a derivative of the respiratory index versus time plot, which gives a slope and therefore direction and magnitude of the change. It may not just be the direction of the change that is important, but also the magnitude (that is, rate of change) which indicates how quickly the change is occurring. A second derivate wrt to time could be used to see the acceleration of change. The second derivative can be displayed numerically and/or graphically.

Going a step further, a change in trend over time could also be considered. For example, a derivative of respiratory index v time is assessed over time, and optionally a second derivative of respiratory index (or some other measure of change in slope/trend) over time is assessed. The first derivative is a trend, and the second derivative is a change of trend over time. The second derivative of respiratory index v time can be assessed against slope and/or magnitude relationship information. It also not necessary to actually obtain derivates versus time, and it is the change over time, however obtained, that is a consideration.

A change in respiratory index over time comprises determining a trend in the respiratory index. Furthermore, the trend could comprise a plurality of trends each themselves changing over time. Each trend becomes an instantaneous trend, specifying the trend (change over time) of the respiratory index at that point in time, and determining a trend could comprise determining a plurality of instantaneous trends over time.. Each trend or instantaneous trend can be represented with a trend parameter comprising magnitude (itself indicating rate of change over time of the respiratory index) and a direction (of the change), and optionally could be in the form of:
a vector, or
a slope (and optionally a magnitude).

A generic respiratory index versus time plot is shown in Figure 2 along with a generic respiratory index threshold. If the respiratory index goes above the threshold, this indicates a good respiratory status (normal respiration, or only mild distress and/or low risk of respiratory failure), whereas if the respiratory index goes below the threshold, this indicates a bad respiratory status ( e.g. deteriorating respiration, respiratory distress and/or thigh-risk of respiratory failure).

Referring to Figure 2, it can be seen that the patient starts at "A" with a patient respiratory status with a low respiratory index, which on its own indicates respiratory distress, such as a high risk of respiratory failure. However, referring to part "A" of the graph, as can be seen from the slope and direction of vectors (trend parameters) of the plot, and from the direction of the plot generally, the respiratory index is trending up towards the threshold, which means the patient is improving. Therefore, in that situation a patient that is on escalated respiratory support (because they are in a high-risk patient respiratory status) may be brought off and de-escalated sooner, or the clinician may at least prepare for de-escalating respiratory support - even though they are still in a high-risk patient respiratory status. Also, in the situation where the patient is not on escalated respiratory support, the clinician may decide not to escalate the respiratory support, even though the patient is below the threshold, on the basis that they are trending towards the threshold, that is, respiratory status is improving.

In contrast, referring to part B of the graph, as can be seen from the slope and direction of the vectors of the plot, and from the direction of the plot generally, the respiratory index is trending down towards the threshold, which means the patient is deteriorating. Therefore, in that situation patient that is on de-escalated respiratory support (because they are in the low-risk patient status) may be put on escalated respiratory support sooner, or the clinician may at least prepare for escalating respiratory support (even though they are still in low-risk patient respiratory status). Also, in the situation where the patient is on escalated respiratory support, the clinician may decide not to de-escalate the respiratory support, even though the patient is above the threshold, on the basis that they are trending downwards towards the threshold.

Various other trends and thresholds could be used in such an analysis, for assessing patient respiratory status and whether to change respiratory support. Using trends provides a better level of information, including some predictive information that assists in reacting and providing the appropriate changes in respiratory support before it is actually needed.

Information relating to respiratory index the change in respiratory index over time, respiratory status and/or changes in respiratory support can all be conveyed in a suitable manner through an IO interface to enable a clinician to assess and determine respiratory support required. The information can also be transferred as required to devices, respiratory support apparatus, and/or servers 100 or the like as required.

### 2.3 Embodiment 2 - assessment method - ROX index trend

Referring to Figures 1, 4, 5, in a possible embodiment of using a respiratory index over time, a change in ROX index over time is used to assess patient respiratory status.

The ROX index is determined as previously described, for example from the calculation of the following. $ROX = \left(SpO2/fiO2\right) / RR$

As noted previously, ROX index can be calculated and used to determine success of respiratory support

The benefit of this approach over the single value approach can be demonstrated from the table below

### Patient 1

| *No* | *Date*/*Time* | *SPO₂(%)* | *FIO₂* | *RR(min-1)* | *ROX* |
|---|---|---|---|---|---|
| *1* | *initiation* | *95* | *0.70* | *34* | *4.0* |
| *2* | *2* | *95* | *0.60* | *32* | *5.0* |
| *3* | *6* | *95* | *0.50* | *32* | *6.0* |
| *4* | *12* | *95* | *0.45* | *30* | *7.0* |

### Patient 2

**Table 1**

| *No* | *Date*/*Time* | *SPO₂(%)* | *FIO₂* | *RR(min-1)* | *ROX* |
|---|---|---|---|---|---|
| *1* | *initiation* | *95* | *0.75* | *32* | *4.0* |
| *2* | *2* | *95* | *0.80* | *34* | *3.5* |
| *3* | *6* | *95* | *0.85* | *37* | *3.0* |
| *4* | *12* | - | - | - | - |

For example, two patients begin NHF treatment and both have a ROX value of 4.0. Because this is only the start of the therapy, the ROX value can be monitored to see whether the index improves. During the first 6 hours, the first patient has a decrease in respiratory rate and the FiO₂ has been lowered, patient 2 has an increase in respiratory rate and the FiO₂ has been increased. As a result, the ROX value at 6 hours for patient 1 is 6.0 and patient 2 is 3.0. Patient 1 has a high likelihood of NHF therapy success and can be maintained on NHF. However, patient 2 has a trending decline and low ROX; therefore escalation of care should be considered.

At the starting point, both patient 1 and patient 2 have a ROX index of 4.0. This is below 4.88 and therefore an indication of risk of respiratory failure. In both those cases, under a single value ROX index assessment, the assessment would be that the patient's respiratory status indicates a high risk of respiratory failure, and an escalation of respiratory support is required. However, it can be seen patient one actually improves soon afterwards - with the ROX index improving to 5.0, 6.0 and 7.0 - well above the 4.88 threshold. As such, any escalation of respiratory support would be premature, and may unnecessarily risk the health of the patient for no benefit. Conversely, patient two deteriorates as their ROX index dropped to 3.5, 3.0 and then goes into respiratory failure. If the FiO2 and respiratory rate increase, the ROX begins to drop in value. An increase in FiO2 and respiratory rate is indicative of the patient deteriorating. The continuous monitoring of the ROX index will be helpful when the patient is an unstable condition. Clearly, an escalation of respiratory support is justified in the situation. A single value of ROX index assessment would not capture this dynamic situation.

Therefore, rather than comparing a single ROX index to a threshold, the change of ROX index over time (trend parameter) can be determined and from the change of the ROX index over time, preferably with respect to a threshold, an assessment is made as to the patient respiratory status, and from that an assessment made as to the required change in respiratory support.

For example, the change in ROX index over time could show a trend of the ROX index improving, which might lead to a de-escalation of respiratory support; or alternatively deteriorating, which might lead to an escalation of respiratory support.

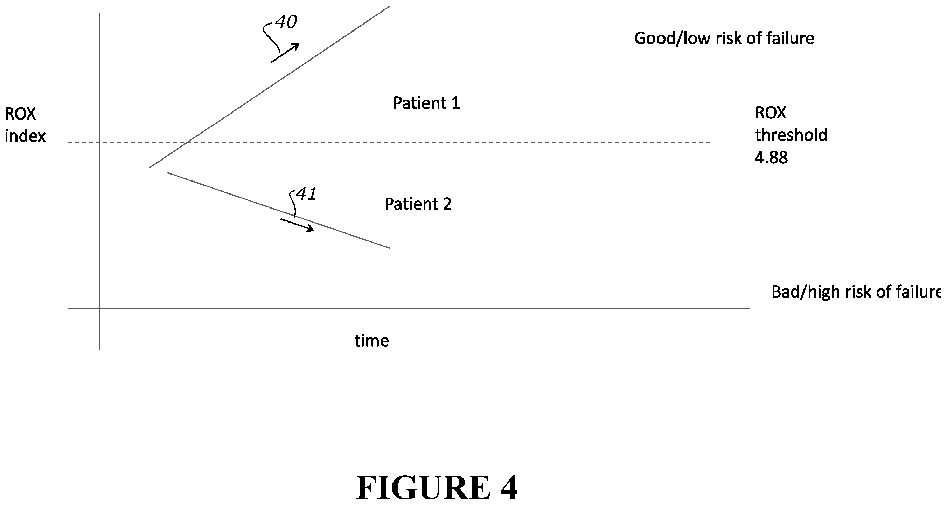

This can be demonstrated with respect to Figures 4 and 5 which show a plot of ROX index versus time, and respiratory rate versus FiO2 respectively for each of the two patients - patient 1 (40), patient 2 (41). Each graph shows the threshold relating to a ROX index of 4.88 which delineates between a patient respiratory status that indicates a high risk of respiratory failure and one that indicates a low risk of respiratory failure. ROX (or other respiratory index) assessment is also demonstrated with respect to Figure 1 which shows a flow diagram of the assessment method.

SpO2, FiO2 and respiratory rate are obtained in the usual way, e.g. from sensors, and the ROX index calculated from that, e.g. in a controller. As previously noted, the apparatus for carrying out the assessment method will be described in more detail below. The ROX index is calculated over time. It might be calculated continuously, or periodically in a suitable manner, and the value stored by the controller.

Furthermore, the change of the ROX index over time is determined in a suitable manner. This can be by way of a trend parameter in the form of e.g. a vector showing the change of ROX index over time, referring to Figure 4, or alternatively by plotting the change of respiratory rate versus FiO2 at various time points, as shown in Figure 5 both these give a measure of magnitude and direction of change. Figure 4 gives it in the form of a slope, while Figure 5 gives it in the form of a vector. Note, these Figures might be provided on a mobile device and/or respiratory apparatus, as described in embodiments below.

Going a step further, a change in trend over time could also be considered. For example, a derivative of respiratory index v time is assessed over time, and optionally a second derivative of respiratory index over time is assessed. The first derivative is a trend, and the second derivative is a change of trend over time. The second derivative of respiratory index v time can be assessed against slope and/or magnitude relationship information.

For example, the trend could comprise a plurality of instantaneous trends, and determining a trend could comprise determining a plurality of instantaneous trends over time. Each trend or instantaneous trend can be represented for example as a vector comprising magnitude and a direction. The instantaneous trend parameters provide an updated trend over time. Figure 5 shows vectors for patient 1 (40) and patient 2 (41) at different times, taken from the data in the table above.

Any other suitable depiction or characterisation of the change in ROX index could be made instead, and these two graphs are by way of example only. The graphs and Figures 5 and 6 are for exemplary purposes only to demonstrate the concept. It may not be necessary for the controller to actually determine and/or display the graphs as above. Rather, the assessment could be made by processing of the ROX index values in a suitable way to gain the same information.

The information relating to ROX index the change in ROX index over time, respiratory status and/or changes in respiratory support can all be conveyed (and/or stored) in a suitable manner through an IO interface to enable a clinician to assess and determine respiratory support required. The information can also be transferred as required to devices, respiratory support apparatus and/or servers 100 or the like as required, e.g. through wired or wires transmission, including NFC.

### 3. Respiratory support apparatus and control method

The respiratory index (and optionally respiratory status) assessment method as described in the embodiments herein can be used to determine how best to provide respiratory support. In a preferred embodiment, the respiratory support is in the form of nasal/tracheal high flow respiratory support using an appropriate apparatus. Therefore, the present embodiments also relate to a method of providing and changing respiratory support based on a respiratory status assessment method, and a respiratory apparatus for providing respiratory support - where the respiratory support is provided based on the assessment method.

**Based on** the assessment phase, one or more of the following respiratory support changes can take place. The changes in respiratory support mentioned herein can be implemented by using the respiratory support apparatus differently.

These can be generally split into uses that provide escalation and de-escalation of respiratory support.

### Escalation:

- The high flow respiratory support is continued, but at a higher or lower level. For example, flow, O2 concentration, humidification, flow oscillation and/or other parameters are increased or decreased.
- The patient is transferred to a more invasive respiratory support such as:
   ∘ NIV pressure respiratory support
   ∘ Mechanical (invasive) ventilator respiratory support via intubation

### De-escalation:

- if it is being used, removing invasive respiratory support and returning to the base respiratory support (for example, NIV respiratory support or nasal high flow), or
- if it is being used, removing NIV respiratory support and returning to the base respiratory support (for example, nasal high flow), or
- if it is being used, de-escalating nasal high flow).

Escalation and/or de-escalation can be trigger automatically and/or via messages, alerts or other indicators to a clinician to indicate a change should be made to respiratory support based on an assessment of respiratory index indicating the patient status is getting better or worse (such as going into or coming out of respiratory distress and/or failure)

In one example, flow rate is changed based on or relative to the change in the respiratory index or to the change in trend.

Where the respiratory apparatus controls change in respiratory support, it might receive instructions or information (e.g. respiratory index, respiratory status, trend parameter or the like) from an assessment device to make the change, or determine the change required then make the change. Where the clinician controls change in respiratory support, they might receive instructions or information (e.g. respiratory index, respiratory status, trend parameter or the like) from an assessment device to make the change, or determine the change required from that information to make the change.

Referring to Figure 6, a high flow therapy respiratory support apparatus that can be used to provide the respiratory support based on the assessment, and/or can implement the assessment method will now be described. Where escalation or de-escalation comprises altering the high flow respiratory apparatus, this can be arranged by the clinician manually and/or the apparatus can be configured to automatically make the change. Where escalation requires mechanical ventilation or NIV pressure respiratory support, the clinician will provide that using an appropriate apparatus.

Figure 6 shows a respiratory support apparatus 10 for providing high flow respiratory support to a patient. The apparatus is configured for delivering apparatus gas flow comprising air and auxiliary gas such as oxygen at a particular fraction. The apparatus 10 could be an integrated or a separate-component-based arrangement, generally shown in the dotted box in Figure 6. In some configurations, the apparatus could be a modular arrangement of components. As such, the apparatus could be referred to as a "system", but the terms can be used interchangeably without limitation. Hereinafter it will be referred to as an apparatus, but this should not be considered limiting. The apparatus is shown as a nasal high flow respiratory apparatus 10, but could become a tracheal high flow respiratory apparatus with a trachea user interface.

The apparatus comprises a flow source 50 for providing a high flow gas 31 such as oxygen or air, or a mix of air and oxygen, and/or one or more other gases. Alternatively, the apparatus can have a connection for coupling to a flow source. As such, the flow source might be considered to form part of the apparatus or be separate to it, depending on context, or even part of the flow source forms part of the apparatus, and part of the flow source fall outside the apparatus.

The flow source could be an in-wall supply of oxygen, a tank of oxygen 50A, a tank of other gas and/or a high flow therapy apparatus with a blower/flow generator 50B. Figure 6 shows a flow source 50 with a flow generator 50B, with an optional air inlet 50C and optional connection to an O2 source (such as tank or O2 generator) 50A via a shut off valve and/or regulator and/or other gas flow control 50D, but this is just one option. A flow source inlet could be termed a supplementary gases inlet. The description from here can refer to either embodiment. The flow source could be one or a combination of a flow generator, O2 source, air source as described. The flow source 50 is shown as part of the apparatus 10, although in the case of an external oxygen tank or in-wall source, it may be considered a separate component, in which case the apparatus has a connection port to connect to such flow source. The flow source provides a (preferably high) flow of gas that can be delivered to a patient via a delivery conduit, and patient interface 51. Depending on the end-use, the patient interface 51 may be an unsealed (also termed "non-sealing") interface (for example when used in high flow therapy) such as a nasal interface (cannula), or a sealed interface (for example when used in CPAP) such as a nasal mask, full face mask, or nasal pillows. The apparatus can also be used with a trachea interface for tracheal high flow to the patient. The patient interface 51 is preferably a non-sealing patient interface which would for example help to prevent barotrauma (e.g. tissue damage to the lungs or other organs of the respiratory apparatus due to difference in pressure relative to the atmosphere). The patient interface may be a nasal interface (cannula) with a manifold and nasal prongs, and/or a face mask, and/or a nasal pillows mask, and/or a nasal mask, and/or a tracheostomy interface, or any other suitable type of patient interface. The flow source could provide a therapeutic gas flow rate of between, e.g. about 0.5 litres/min and about 375 litres/min, or any range within that range, or even ranges with higher or lower limits. Possibly flow ranges are described in more detail in the definition of terms section above.

The flow rate may comprise a therapeutic flow rate component, wherein the therapeutic flow rate is about 375 litres/min to about 0 litres/min, or about 150 litres/min to about 0 litres/min, or is preferably about 120 litres/min to about 15 litres/min, or is more preferably about 90 litres/min to about 30 litres/min.

A humidifier 52 can optionally be provided between the flow source 50 and the patient to provide humidification of the delivered gas. This humidifier can comprise, for example, a heater plate, a region to receive a humidifier chamber (tub), and a humidifier chamber for holding water. This could be a humidifier integrated with the flow source 10 to form an integrated apparatus 59 (see dotted lines) or separate but attachable to the flow source 10. Alternatively, the humidifier 52 could be a standalone humidifier with a chamber and base, where the humidifier is coupled to the flow source 10 via conduits or other suitable means. One or more sensors 53A, 53B, 53C, 53D such as flow rate, oxygen fraction or other gas fraction, full or partial pressure, humidity, temperature or other sensors can be placed throughout the apparatus and/or at, on or near the patient 16. Alternatively, or additionally, sensors from which such parameters can be derived could be used. In addition, or alternatively, the sensors 53A-53D can be one or more physiological sensors for sensing patient physiological parameters such as, heart rate, oxygen saturation (e.g. pulse oximeter sensor 54E), partial pressure of oxygen in the blood, respiratory rate, FiO2, partial pressure of O2 and/or CO2 in the blood. Alternatively, or additionally, sensors from which such parameters can be derived could be used. Other patient sensors could comprise EEG sensors, torso bands to detect breathing, and any other suitable sensors. The sensors could be considered part of the apparatus or separate to it, depending on where they are positioned. E.g., and without limitation, the physiological sensors might be considered separate to the apparatus, whereas the sensors for measuring parameters of the apparatus might be considered part of it. In some configurations the humidifier may be optional, or it may be preferred due to the advantages of humidified gases helping to maintain the condition of the airways. Humidification is preferably used with high flow gas flows to increase patient comfort, compliance, support and and/or safety. One or more of the sensors might form part of the apparatus, or be external thereto, with the apparatus having inputs for any external sensors. Sensors could be non-invasive.

In some configurations, there is at least one ultrasonic sensor. This is advantageous because it is a fast acting sensor that provides fast reading of O2, for FiO2 or FdO2 readings. An ultrasonic sensor allows better and more accurate control of fraction of O2 due to speed to response.

In some configurations, the SpO2 sensor could be wireless.

The output from the sensors is sent to a controller to assist control of the apparatus, including among other things, to vary gas flow and/or oxygen fraction. This enables change of use of the apparatus in accordance with the assessment. The controller is coupled to the flow source, humidifier and sensors. It controls these and other aspects of the apparatus to be described below. The controller can operate the flow source to provide the delivered flow of gas. It can also operate the gas flow modulator(s) (including the flow source) to control the flow rate, pressure, volume, O2 fraction and/or other parameters of gas provided by the flow source based on feedback from sensors, or optionally without feedback (e.g. using default settings). The controller can also control any other suitable parameters of the flow source to meet or increase oxygenation requirements and/or CO2 removal. The controller 19 can also control the humidifier 52 based on feed-back from the sensors 53A-53D, 14. Using input from the sensors, the controller can determine oxygenation requirements and provide information to a medical professional (who may control the components of the respiratory apparatus to provide the desired therapy, e.g. flow rate, O2 fraction, humidity, etc.) and/or control parameters of the flow source, gas flow modulator(s) and/or humidifier as required. Alternatively, the embodiments could be provided as a standalone monitoring apparatus, independent of a respiratory apparatus that provides information to a medical professional and/or communicate and control components of the respiratory apparatus to provide a desired therapy. The medical professional can then control the respiratory apparatus to provide the desired therapy. Accordingly, the controller may not always determine oxygenation requirements and control parameters of the apparatus.

The controller 19 is also configured to operate the apparatus so that the apparatus gas flow has flow rate that provides a flow rate as described. It can also operate the flow source to control the flow, pressure, volume and/or other parameters of gas provided by the flow source based on feedback from sensors, or optionally without feedback (e.g. using default settings). The controller can also control any other suitable parameters of the flow source to meet oxygenation requirements.

The controller 19 is also configured to operate the apparatus so that the apparatus gas flow has a gas proportion (such as O2 fraction or other gas fraction) that provides gas proportion (such as gas fraction and/or gas partial pressure) as described. It can do this through any suitable means such as controlling a proportional valve coupled to an O2 source 50A or any other means previously described. In an embodiment , and single proportional valve is used prior to an impeller, which controls a O2 fraction into the inlet of the impeller along with the ambient air, and the impeller controls the flow rate. The controller 19 can control the proportional valves to operate as required to achieve the gas proportion as described herein.

An input/output interface 54 (such as a display and/or input device) is provided. The IO interface is for receiving information from a user (e.g. clinician or patient) that can be used for determining oxygenation requirements. The IO interface can comprise, for example, a display and input device(s) such as button(s), a touch screen, or the like. The screen may be a detachable screen. It can display numerical and/or graphical information (such as 2D/3D plots).

The controller can also be configured to determine and implement the assessment method as previously described based on input from e.g. sensors (from the apparatus itself and/or a patient) and other information, such as preconfigured information and/or information entered via the I/O interface.

The nasal high flow respiratory support apparatus is controlled in the usual manner known to those in the art to provide a flow rate to the patient and/or control oxygen fraction, among providing other operational parameters to provide respiratory support.

The nasal high flow therapy respiratory support apparatus is configured to try to control to the predetermined concentration of peripheral arterial oxyhaemoglobin i.e. 95% SpO2. Typically SpO2 in patients with hypoxemic respiratory failure is controlled to 92 -96%. In patients with hypercapnia SpO2 is typically controlled to 88 - 92% The NHF device is configured to control the oxygen fraction FiO2 to try and achieve the set SpO2. The device measures the FiO2 delivered and measures respiratory rate (RR) of the patient.

In addition, the nasal high flow respiratory support apparatus can be operated to change the respiratory support when it is determined by the assessment method (as per the embodiments above) that a change in respiratory support is required. This will now be described.

The respiratory support apparatus can have a communication module for communication to a separate assessment apparatus, where applicable - see below. The communication module can include WiFi module, a Bluetooth module, mobile telecommunications module (such as GSM module) and/or an NFC communication module. The NFC communication module comprises a coil and associated processor that is configured to allow NFC communication of data.

The respiratory apparatus has and/or connects to sensors that provide
- physiological parameters, (which can comprise respiratory parameters), and
- respiratory apparatus parameters (which can comprise operational parameters)
to obtain parameters for operation of the apparatus (such as temperature, humidity, pressure, flow sensors) and parameters to determine the respiratory index (such as SpO2, FiO2 (or FdO2) Respiratory Rate).

To provide change in respiratory support, the apparatus can control itself or be controlled by a clinician to continue high flow respiratory support, but at a higher or lower level. For example, flow, O2 concentration, humidification, flow oscillation and/or other parameters are increased or decreased. Any of the respiratory support changes described herein in any of the embodiments relevant to the respiratory apparatus can be made.

### 4. Assessment apparatus

Embodiments of the apparatus for implementing the assessment method will now be described.

### 4.1 Embodiment 1 - assessment apparatus part of respiratory support apparatus

In one embodiment, the respiratory support apparatus implements any assessment method described herein. Preferably, the controller of the respiratory support apparatus as shown in Figure 6 is used. It is preconfigured with any data and/or instructions required to carry out the method, and can receive the required input from sensors, the user input, and any other sources. This provides the advantage of integrated sensors or inputs for sensors which enables non-invasive monitoring and have a single device that can measure the various parameters and provide/change respiratory support required.

The controller is programmed to calculate the respiratory index using a suitable equation such as one of those above, the preferred one being ROX index. The equations for respiratory index including ROX index have been stated earlier.

Alternative means of determining the respiratory index could be used, such as a lookup table, database, or similar which correlates input data to the appropriate respiratory index. From this point onwards, the apparatus will be described with reference to calculation of an ROX index using the equation above, but this should not be considered limiting and any of the description below could be equally applied to determination of another respiratory index, be it calculated or otherwise determined, based on receiving the appropriate input parameters.

Reference will be made to Figure 1 which shows the operation of the controller and apparatus. This has parallels to the flow diagrams above describing the assessment method, but the present diagrams are focused on the actual actions taken by the controller. Referring to the flow diagram in Figure 1 the controller receives the various physiological and/or operational parameters, such as respiratory rate, SpO2, FiO2 from the appropriate sensors.

Next, the controller calculates the ROX index and ROX index change over time using these input parameters. In the case of the dynamic assessment, the ROX index is calculated continuously or periodically to provide a sequence of values that can be used for trend assessment as shown in Figure 4. Where appropriate, as described in the embodiments above, the apparatus could determine the respiratory index change over time to get a trend parameter or a plurality of trend parameters.

The patient respiratory status is then optionally assessed, by processing the ROX index values change over time and/or trend parameters. Assessing the patient respiratory status might not be a step in itself, but rather the outcome of a range of steps. The outcome might be an actual determination of patient respiratory status, or simply some information which could relate to a patient respiratory status but is not actually determined. But the information can be used to assess the change in respiratory support required which is consistent with improving the patient outcome based on what the patient respiratory status would be if it were actually specifically determined. However, for explanatory purposes reference to assessing a patient respiratory status will be made. Alternatively, the information could be obtained by processing the ROX index values change over time and/or trend parameters directly.

In the case of a dynamic assessment of a continuously or periodically determined respiratory index, the controller determines a trend parameter (or plurality of trend parameters) of the respiratory index over time to provide magnitude (amount of rate of change) and direction of change. For example, time series measurements are taken and a trend is determined based on the change in subsequent index calculations. That along with threshold information can be used to provide an assessment of respiratory status, e.g. distress and/or a change in respiratory state or distress and/or a trend in the respiratory state or distress (e.g. deterioration) For example, the high or low risk of respiratory failure could be determined, as previously described.

In general terms, where there is respiratory distress and/or deterioration of patient respiratory status (such as a risk of respiratory failure), an escalation of respiratory support is provided, and where there is no respiratory distress and/or improvement of respiratory status (such as no of respiratory failure) de-escalation is provided. However, this was all explained further in detail with respect to the assessment methods described earlier. The action taken should not be limited to specific respiratory status/trends, and these are examples only. Determined actions based on respiratory index or respiratory index change/trend (which might correspond to particular respiratory statuses or trends) are used.

If it is determined that an escalation of respiratory support is required, the controller may also determine what type of escalation, which can be one or more of:
- The high flow respiratory support is continued, but at a higher level. For example, flow, O2 concentration, humidification, flow oscillation and/or other parameters are increased or decreased
- The patient is moved to a more invasive respiratory support such as:
   ∘ NIV pressure respiratory support
   ∘ Mechanical ventilator respiratory support via intubation.

Depending on which of the escalation methods are required, the apparatus can do one or more of the following:
- controller controls apparatus to escalate respiratory support, and/or
- the apparatus conveys information, in the form of instructions, status, alarms or the like, advising the clinician to escalate respiratory support.

Information can also be provided on the I/O interface e.g. a display screen to inform and/or instruct the clinician. For example, the graphs of Figures 5 and 6 might be displayed. The screen can be detachable which means the screen can be moved to eye level.

The controller continually repeats the method to continually assess patient respiratory index (an/or status) and continually adjust the operation of the respiratory support apparatus accordingly and/or continually convey information to the clinician regarding escalation and/or de-escalation of respiratory support.

The respiratory support apparatus can have a communication module for communication to a separate assessment apparatus, where applicable - see below. The communication module can include WiFi module, a Bluetooth module, mobile telecommunications module and/or an NFC communication module. The NFC communication module comprises a coil and associated processor that is configured to allow NFC communication of data.

Some additional operational details of the respiratory support apparatus will now be described. The apparatus preferably uses a start-up period. The start-up period allows a patient to acclimatise to the high flow respiratory support provided to the patient. Further a baseline respiratory index value can be established during the start-up period. This is a baseline index. The start-up period can be between 30 mins to 3 hours. Preferably it is between 1 to 2 hours of initial readings and acclimatising to the therapy.

The apparatus can have an update period. The update period allows the respiratory support apparatus to take measurements and calculate a new respiratory index (e.g. ROX index value) and calculate a vector (or other trend parameter). The vectors are calculated between two consecutive ROX index value calculations. The update period may be between 5 mins and 30 mins or any other period. Respiratory support apparatus preferably takes measurements of respiratory rate and FiO2. These can be transmitted to the mobile device or stored in the device. The sampling period can be between 10 seconds to 20 mins.

The flow rate changes may be a smooth transition or a continuous change. Alternatively, the flow rate changes may be step changes based on the trend of respiratory index. As ROX changes, flow is step changed in then update period. ROX is calculated after the flow rate is changed during the update period. The flow is changed until a threshold, at which point it is uncomfortable.

As a further alternative, the respiratory support apparatus may not automatically change flow rate. Instructions to the clinician are made from the assessment apparatus to change the flow based on the respiratory index. The instructions may include a video or a series of images that illustrate how to change the flow rate and how much to change the flow rate by.

### 4.2 Embodiment 2 - assessment apparatus separate to respiratory support apparatus

Referring to Figure 3, in an alternative embodiment, the assessment method is carried out in an apparatus separate 20 to the respiratory apparatus. The assessment apparatus is in communication with the respiratory apparatus, sensors and/or the patient. The assessment apparatus can operate in much the same way to make the assessment as described previously with respect to Figure 1 and the respiratory apparatus, albeit with some differences. In such an embodiment, the separate assessment apparatus and respiratory support apparatus and/or the sensors could form a respiratory assessment and support system.

The assessment apparatus has a controller. The controller is programmed to calculate the respiratory index using a suitable equation such as one of those above, the preferred one being ROX index. The respiratory index equations above can be used, or a lookup table, database, or similar as described above.

Reference will be made to Figure 1 which shows the operation of the controller and apparatus. This has parallels to the flow diagrams above describing the assessment method, but the present diagrams are focused on the actual actions taken by the controller. Referring to the flow diagram in Figure 1 the controller receives the various physiological and/or operational parameters, such as respiratory rate, SpO2, FiO2 from the appropriate sensors.

Next, the controller calculates the ROX index and ROX index change over time using these input parameters. In the case of the dynamic assessment, the ROX index is calculated continuously or periodically to provide a sequence of values that can be used for assessment as shown in Figure 1. Where appropriate, as described in the embodiments above, the apparatus could determine the respiratory index change over time to get a trend parameter or a plurality of trend parameters.

The controller is preconfigured with any data and/or instructions required to carry out the method, and can receive the required input from the respiratory apparatus, sensors, user input and/or any other sources.

Once the controller has the required information, it can assess the patient respiratory status as previous described with reference to Figure 1 in the methods and/or as described for the respiratory support apparatus.

Once the assessment of respiratory status and/or the respiratory support response has been made the apparatus can display the appropriate actions and/or communicate with the respiratory apparatus and/or clinician to provide instructions and/or information for the controller to determine and/or action appropriate respiratory support change buy the controller and/or clinician

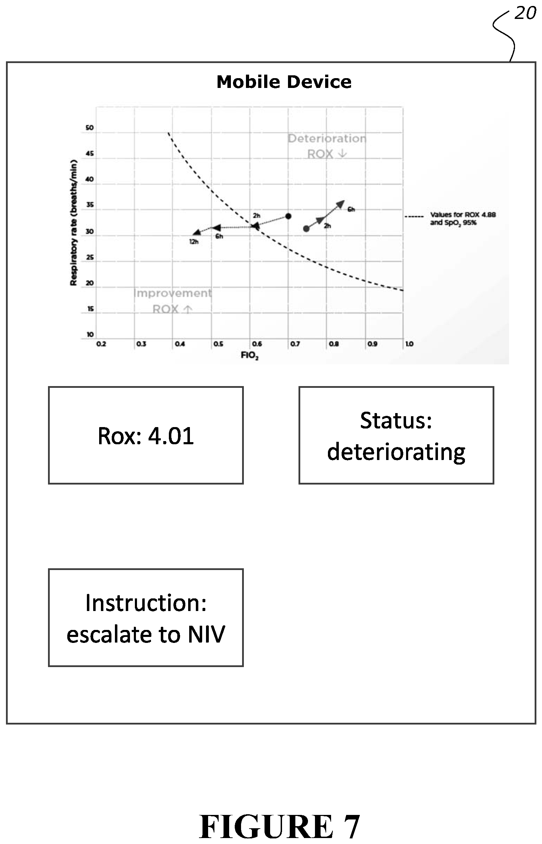

In one particular non-limiting embodiment, the assessment apparatus takes the form of a mobile device such as a smart phone 20, tablet or other portable and/or mobile communication device, such as shown in Figure 7. The mobile device is running an assessment app on a controller. It has an IO interface for presenting information, including alarms

The mobile device communicates with the respiratory support apparatus as described above and is programmed with an app that carries out the assessment method. In this embodiment ROX index is used, although it will be appreciated that the same technology could be configured to work with any other respiratory index as described above. The mobile communication device comprises an app that receives inputs of respiratory rate, FiO2 and the SpO2 set point (or alternatively actual measured SpO2) from the clinician, and/or received from the respiratory support apparatus and/or a sensors (e.g. SpO2 sensor or as otherwise described earlier - wearable or otherwise), e.g. via BluetoothTM, NFC, or other wireless or wire communication modes. A healthcare provider with a portable device like a phone or a tablet can additionally or alternatively download the data via NFC using and an application that allows a quick a secure downloading of the data. The inputs are input or prompted at time intervals. The mobile device calculates the ROX index value at that time based on the inputs. Further the mobile communications device app, calculates the ROX index trend parameter (e.g. a vector) based on the input from the user. Further the mobile device can calculate a plurality of such trend parameter vectors over time, each being an instantaneous vector, and each indicating the ROX index change trend at that point in time. The vectors are illustrated on a I/O interface of the mobile device, such as shown in Figures 5, 7. This shows vectors at various time points, 2 hours, 6 hours and 12 hours showing the change in ROX index.

The app/mobile communications device provides an assessment tool (diagnostic tool) for the clinician to quickly determine a change in the patient's condition based on the change of ROX index and the trend of the change of the ROX index over time. This visual plot of the ROX index vector such as shown in Figure 5 allows a clinician to make an objective decision about a patient's condition and allows a clinician to escalate or de-escalate the patient respiratory support earlier. The earlier the patient is escalated if ROX index vector trend is the direction of deterioration, the better outcome could be resulting in reduced mortality due to earlier intervention and escalation to mechanical ventilation.

The earlier the patient can be de-escalated the ROX index trend is in the direction of improvement, the less risk patient faces from unnecessarily escalated respiratory support.

As an alternative, the assessment made by the app can be communicated to the respiratory support apparatus and the respiratory support apparatus can make the appropriate change and respiratory support.

The clinician can determine the change in respiratory support required. Alternatively, the app can also determine the change in respiratory support that is appropriate, and either provide this to the respiratory apparatus and/or convey this to the clinician via the mobile communications device. As previously noted, this change in respiratory support could be any one or more of the following implemented by the clinician and/or the respiratory apparatus.
- The high flow respiratory support is continued, but at a higher level. For example, flow, O2 concentration, humidification, flow oscillation and/or other parameters are increased or decreased
- The patient is moved to a more invasive respiratory support such as:
   ∘ NIV pressure respiratory support
   ∘ Mechanical ventilator respiratory support via intubation.

In one example, flow rate is changed based on or relative to the change in the respiratory index or to the change in trend. For example, the flow change can be proportional to the gradient of the respiratory index change vector.

Depending on which of the escalation methods are required, the apparatus can do one or more of the following:
- controller controls the respiratory apparatus to escalate respiratory support, and/or
- the apparatus conveys information, in the form of instructions, status, alarms or the like, advising the clinician to escalate respiratory support.

For example, it might be assessed that the flow rate provided to the patient via the respiratory apparatus needs to change. The mobile communications app is configured to calculate a required change in the flow rate provided to the patient. The app is configured to calculate a new set flow rate based on either the ROX index or a vector of the ROX index (i.e. a trend of change of the ROX index), and/or some other parameter or parameters. The app provides instructions to the clinician via the I/O interface of the mobile communications device with information of the new flow rate or a change in flow rate required away from an initial set flow rate. Alternatively, the information can be communicated directly to the respiratory apparatus which will The set flow rate data is also transmitted from the NHF device to the phone via NFC or Bluetooth as part of the phone interacting with the device.

The separate assessment device can communication with the respiratory support apparatus via the communication module of the respiratory support apparatus. The communication interface can include WiFi module, a Bluetooth module and an NFC communication module. The NFC communication module comprises a coil and associated processor that is configured to allow NFC communication of data.

In an alternative some of the assessment method is carried out partially in the separate apparatus and partially in the respiratory support apparatus
Alternatively, the plot and ROX vectors can be calculated in the NHF device and displayed on a I/O interface of the NHF device.

### 5. Exemplary embodiment using ROX index and separate assessment device

The present method and apparatus embodiments described herein can be combined in any suitable order to provide an apparatus and/or system to provide respiratory assessment and support.

### 5.1 Exemplary example with use case

Referring to apparatus Figures 3, 6 and 7, and flow diagram of Figure 1 one non-limiting exemplary example is described here.

It implements respiratory assessment using a mobile telecommunications device with an app utilising a change (trend) in ROX index (preferably a change/trend over time), and a nasal high flow respiratory support apparatus which is controlled at least in part based on the assessment of ROX index trend. Alternatively, a NIV apparatus could be used.

The ROX index is calculated and used to determine success of high flow respiratory support - such as nasal or tracheal high flow respiratory support. The change in ROX index over time is used to predict if a patient is going to improve or deteriorate. In particular, the disclosure is directed to determining a temporal change in ROX index values or using a vector or vectors of ROX index over time to determine how the ROX index is changing and providing an indication if a patient is deteriorating or if a patient is improving. If the FiO2 and respiratory rate increase, the ROX index begins to drop in value. An increase in FiO2 and respiratory rate is indicative of the patient deteriorating. The continuous monitoring of the ROX index will be helpful when the patient is an unstable condition. The nasal high flow device is controlled to amend flow rate provided to the patient and/or control oxygen fraction. The nasal high flow respiratory support apparatus is configured to try and control to the predetermined concentration of peripheral arterial oxyhaemoglobin (i.e. 95% SpO2). Typically, SpO2 in patients with hypoxemic respiratory failure is controlled to 92 -96%. In patients with hypercapnia SpO2 is typically controlled to 88 - 92%. The NHF device is configured to control the oxygen fraction FiO2 to try and achieve the set SpO2. The device measures the FiO2 delivered and measures respiratory rate (RR) of the patient.

A mobile device. E.g. smartphone or tablet 20, is used by e.g. a clinician - see Figure 7. The mobile device of Figure 7 has the controller and IO interface of the device in Figure 3 and has a IO interface as a touch screen that displays information and allows input of information - e.g. through a touch screen keyboard. It can also have speakers for conveying alarms, prompts, spoken messages and the like.

The mobile device is configured to wirelessly communicate with the respiratory support apparatus such as in Figure 3, 6. This forms a respiratory assessment device and system, such as shown in Figure 3. The mobile device receives required information from sensors as previous described and calculates the ROX index value at that time based on the inputs. Further the mobile communications device app, calculates the ROX index trend parameter (e.g. a vector) based on the input from the user. Further the mobile device can calculate a plurality of such trend parameter vectors over time, each being an instantaneous vector, and each indicating the ROX index change trend at that point in time. The vectors are illustrated on a I/O interface of the mobile device, such as shown in Figure 5, 7. In one example the mobile device is configured to communicate with the NHF device using an NFC protocol. A user can tap the mobile device on the NHF device at a predefined location that is the location of the NFC communication module. Other options for communication are possible.

The mobile communication device comprises an app that is configured to be activated when the mobile device is tapped on the NHF device. Tapping the mobile device on the NHF device causes RR data, FiO2 data and the pre-set SpO2 set point (or actual measured SpO2) to be transmitted to the mobile device. The mobile device is configured to receive inputs of respiratory rate, FiO2 and the SpO2 set point (or alternatively actual measured SpO2) from the clinician, and/or received from the respiratory support apparatus and/or a sensor (e.g. SpO2 sensor), e.g. via Bluetooth^{™}, NFC, or other wireless or wire communication modes. The inputs are input or prompted at time intervals. The respiratory rate, FiO2 data can be measured at predetermined time intervals. The time interval could fall between about 1min to about 2 hours, or any other suitable time interval. In one example measurements are taken every 15 mins. In another example measurements are taken every hour or every 2 hours. Alternatively, or additionally, the app may receive inputs of respiratory rate, FiO2 and the SpO2 set point from the clinician via a I/O interface. The inputs are input or prompted at regular time intervals.

The app is configured to calculate a ROX index value from the data received at each interval. A vector of the ROX index value is calculated based on the respiratory rate and FiO2 at various time intervals. A sum of the ROX index calculations over a period of time e.g. 12 hours is calculated in the phone app. The plot is developed. The app also calculates a change in the ROX index and a trend of the change of ROX index.

An increase in respiratory rate and FiO2 indicates deterioration of the patient's condition. A reduction in respiratory rate and FiO2 indicates an improvement. Further a reduction in FiO2 alone is an indication of improvement. A reduction in respiratory rate is an indication of improvement of the patient.

The app is configured to create the plot shown in Figure 5 and present it on the I/O interface for the user as shown in Figure 7. The ROX index values are plotted and a vector of the ROX index indicative of a change of ROX index over time is plotted. The trend of the ROX index changes are plotted on the plot of respiratory rate vs FiO2.

Referring to Table 1 earlier, raw measurements for patient 1 and patient 2 are shown. The app calculates the ROX index and ROX index vector between various ROX index values at various time intervals for each patient and plots this ROX index, and vectors on a plot. The plot is shown in Figure 5. This plot would be presented to a clinician on the mobile device screen in Figure 7 to allow for quick diagnosis.

A threshold ROX value is plotted as a threshold line, in this example 4.88. The line indicates a successful ROX index - that is it delineates between good patient respiratory status and bad patient respiratory status. A movement or trend of the ROX index toward the upper right is indicative of deterioration of the patient's condition. This movement of the ROX index corresponds to a reduction of ROX index value which also relates to a patient condition deteriorating.

The Figure 5 plot on the screen of the device in Figure 7 shows for patient 1 (40) 3 vectors, each showing the instantaneous trend at hours 2, 6 and 12. Each instantaneous vector shows a magnitude and direction that shows the patient is trending towards (i.e. the vector is pointing towards) a lower ROX value and therefore is improving. Overtime, each vector shows the improvement trend is continuing. The first vector shows the patient is trending towards the ROX threshold 4.88 (below which the risk of respiratory failure is gone or at least significantly less) and by the second 6 hour vector, the patient respiratory rate is similar but the ROX index has dropped below the ROX threshold, which means the risk of respiratory failure has gone or is at least significantly less.

The Figure 5 plot on the screen of the device in Figure 7 shows for patient 2 (41) 2 vectors, each showing the instantaneous trend at hours 2 and 6. Each instantaneous vector shows a magnitude and direction that shows the patient is trending towards (i.e. the vector is pointing towards) a higher ROX value and therefore is deteriorating. Overtime, each vector shows the deterioration trend is continuing. The first and second vectors shows the patient is trending further away from the ROX threshold 4.88 (below which the risk of respiratory failure is gone or at least significantly less), which means the risk of respiratory failure is increasing.

Like in the other embodiments, the device can also display other parameters, such as the ROX index, the patient respiratory status, instructions on what to do (including any respiratory support changes) and any other information through an IO interface. It can also provide audible alarms and/or audible messages conveying the same information. The information can also be transferred (and/or stored) as required to devices, respiratory support apparatus and/or servers 100 or the like as required, e.g. through wired or wires transmission, including NFC.

The app provides a tool for the clinician to quickly determine a change in the patient's condition based on the change of ROX index and the trend of the change of the ROX index over time. This visual plot of the ROX index vector allows a clinician to make an objective decision about a patient's condition and allows a clinician to escalate the patient earlier. The earlier the patient is escalated if ROX index vector trend is the direction of deterioration, the better outcome could be resulting in reduced mortality due to earlier intervention and escalation to mechanical ventilation.

The phone app is configured to calculate a required change in respiratory support. For example, it can determine the flow rate increase to be provided to the patient. The phone app is configured to calculate a new set flow rate based on either the ROX index or a vector of the ROX index (i.e. a trend of change of the ROX index). The phone app provides instructions to the patient via the I/O interface of the mobile device with information of the new flow rate or a change in flow rate required away from an initial set flow rate. The set flow rate data is also transmitted from the NHF device to the phone via NFC or Bluetooth as part of the phone interacting with the device.

Alternatively, the plot and ROX vectors can be calculated in the NHF device and displayed on a I/O interface of the NHF device.

The clinician can determine the change in respiratory support required. Alternatively, the app can also determine the change in respiratory support that is appropriate, and either provide this to the respiratory apparatus and/or convey this to the clinician via the mobile communications device. As previously noted this change in respiratory support could be any one or more of the following implemented by the clinician and/or the respiratory apparatus.
- The high flow respiratory support is continued, but at a higher level. For example, flow (flow rate), O2 concentration, humidification, flow oscillation and/or other parameters are increased or decreased
- The patient is moved to a more invasive respiratory support such as:
   ∘ NIV pressure respiratory support
   ∘ Mechanical ventilator respiratory support via intubation

Depending on which of the escalation methods are required, the apparatus can do one or more of the following:
- controller controls apparatus to escalate respiratory support, and/or
- the apparatus conveys information, in the form of instructions, status, alarms or the like, advising the clinician to escalate respiratory support.

Change in operation of the respiratory support apparatus based on the clinician and/or assessment device is then implemented. The respiratory support apparatus is controlled or controls itself so that the flow based on the change i.e. trend i.e. vector of respiratory index to improve the respiratory index (and/or respiratory status).

In one implementation, the embodiment comprises a mobile device receiving information from wearable sensors. The information is used as above and information conveyed (and/or stored) to the clinician and also a respiratory support device to control the device, e.g. through wired or wires transmission, including NFC.

In one implementation, and referring to Figure 5, a length of the vector is the magnitude. The magnitude determines how big the change of the ROX vector over time is. In one example, the maximum value on the plot for an adult could be approximately at (FiO2 1.0; respiratory rate 45) and the minimum value at approximately (FiO2 0.21; respiratory rate 15). A distance between these points is the maximum magnitude of the vector. The respiratory apparatus can display magnitude, i.e. from the start of therapy, in the form of a bar, dial, colour, %, numbers or the like. If the magnitude is big and the vector movers in the lower left corner (direction) then the therapy is very effective. This can be a simple way to display the therapy effect of the device. The above relates to calculating the magnitude of the ROX index vector i.e. at the instantaneous trend you define the vector with a magnitude and direction. The magnitude is calculated based on FiO2 and respiratory rate. More specifically magnitude = Square root of (FiO2² + RR²). The magnitude and direction is used to define the effectiveness of the therapy. If the magnitude is large and the direction of the vector is moving in the right direction, then therapy is very effective. Magnitude relates to how quickly the patient's health is improving. The direction of the ROX vector may be colour coded. For example, the vector may be illustrated in a first colour if the vector direction illustrates patient respiratory status is improving. The vector may be illustrated in a second colour if the vector direction illustrates a patient respiratory status is deteriorating i.e. getting worse. Referring to Figure 5, if the vector is presented in a first colour if the vector direction is toward the bottom left i.e. an improvement of respiratory status. Alternatively, the vector is illustrated in a second colour if the vector direction is toward the upper right i.e. a deterioration of the patient respiratory status. Further the vector may be presented such that the length corresponds to the magnitude.

The presented information of magnitude and direction is used by a clinician to determine whether to continue with high flow, or if a patient needs to be escalated to a different therapy. Alternatively, the mobile device may alarm if the magnitude is above a threshold and the direction of the vector is trending toward a deterioration of the patient respiratory status. Alternatively, the respiratory support device user interface may be configured to display the vector. The respiratory support device is configured to alarm if the magnitude is above a threshold and the direction of the vector is trending toward a deterioration of the patient respiratory status. The respiratory support device may be configured to automatically increase the flow rate

Some exemplary examples of respiratory support that might be provided (which comprises among other things, although not limited to, changes in respiratory support) based on respiratory index/status assessments are as follows.

These can be applied to any of the embodiments described herein.
- The flow rate provided to the patient is varied. The flow rate may be increased or decreased by a predetermined amount. Alternatively, the flow rate provided to the patient may be varied based on the magnitude of the respiratory index vector. A flow rate increase helps to reduce respiratory rate because it increases expiratory resistance. The additional flow can also increase the amount of oxygen (not an increase in FiO2), but the greater flow rate may improve flushing and increase the O2 amount provided to the lungs. This can help with oxygenation of the patient which can increase the ROX index value.
- The flow (e.g. flow rate) is increased if the respiratory index vector indicates a deterioration of the patient. The flow is increased to a limit away from a base line flow rate. Further the flow may be reduced as the respiratory index vector indicates an improvement in patient health. The reduction may be away from a baseline until a minimum required flow OR flow is reduced away from an increased flow rate value.
- In one example, respiratory index is ROX index which is based on a SpO2 set point, FiO2 and respiratory rate. The respiratory support apparatus may also be configured to control the flow rate based on the change in ROX index.
- If the ROX index drops below the threshold e.g. 4.88 then the flow rate can be increased in order to try and provide additional respiratory support.
- The flow rate may be increased from a base set flow rate to a new flow rate based on the change in the ROX index. Alternatively, the rate of change of the flow rate may be proportional to the rate of change of the ROX index.
- In a further example the NHF device may be configured to control flow rate provided by the NHF device (and hence the motor speed of the blower) based on the change in respiratory rate. For example, if respiratory rate increases the flow rate can be increased to reduce the respiratory rate, and thereby improve ROX index. The flow rate change may be proportional to the change in respiratory rate. Alternatively, the flow rate change may be a function of the change in respiratory rate or a function of the respiratory rate. The flow rate is increased if the respiratory rate increases to provide additional expiratory pressure i.e. additional resistance to breathing out such that the user's respiratory rate is slowed.
- However, if the ROX index vector trends toward the safe level and above the safe level i.e. toward a threshold that indicates deterioration, the NHF device may alarm to indicate the patient needs to be escalated.
- The respiratory support apparatus may also change the flow rate based on the change in FiO2 requirements. If the FiO2 requirements to meet the required SpO2 increases, the flow rate may be increased. The increase in flow rate is proportional to the increase in FiO2 or may be a function of the increase in FiO2. The increased flow rate works to reduce respiratory rate and increases the overall amount of O2 delivered. This can help to reduce the requirements of FiO2 and cause a change in the ROX vector toward a safe direction. The flow rate increase may be in increments of 2L/min to 10L/min from a base flow rate of 30 L/min. If a max flow limit is reached, then the respiratory support apparatus may alarm.
- Further to the above flow control, the respiratory support apparatus may provide breath synchronized flow. The flow rate delivered during expiration is lower than the flow rate during inspiration. The patient's breathing phase is detected using a pressure sensor, or flow sensor or a combination thereof. One example is an integrated pressure sensor in the patient interface. Another example is a flow sensor integrated into the patient interface. A further example is calculating a change in flow or a resistance to flow based on a pressure sensor of flow sensor (integrated into the respiratory support apparatus).
- In one example the inspiratory flow rate and expiratory flow rate provided to the patient are predefined e.g. by the clinician or predefined by a patient who selects the level of "comfort". The level of comfort defines the difference between the expiratory flow rate and inspiratory flow rate. E.g. comfort level 1 = exp flow rate is -10L/min from inspiratory flow rate. Comfort level 2 = exp flow rate is -20 L/min etc.
- The flow controller preferably uses feedback control to control the flow rate and switch flow rate between the inspiratory flow rate value and expiratory flow rate value.
- If the ROX index (i.e. respiratory index) trend (vector) is trending to indicate deterioration of the patient, the expiratory flow rate (i.e. flow rate during expiration) is increased away from a predefined expiratory flow rate.
- The increased flow rate (e.g. increased constant flow rate or increased expiratory flow rate) increases the expiratory impedance. The increased expiratory impedance makes it more difficult for a patient to breathe out. This reduces the respiratory rate of the patient thereby improving the respiratory index (e.g. ROX index).
- Further increasing the flow rate provided during expiration can also help to improve flushing of the upper airways and improve CO2 clearance from the airways. this can improve oxygenation of the patient and reduce FiO2 requirement or make oxygenation more efficient. This can help to improve the respiratory index or change it to indicate improvement of the patient's health condition. The increased flow rate also ensures the flow rate delivered is greater than or equal to inspiratory demand, thereby reducing the entrainment of ambient air. Reducing ambient air entrainment ensures FiO2 concentration is more consistent.
- The mobile device is configured to determine a change in flow provided by the respiratory apparatus required to improve the respiratory index, and presenting on the IO interface of the mobile device instructions to change flow rate. For example, the flow rate is increased to improve the respiratory index.
- An O2 valve of the respiratory apparatus may be controlled to either increase FiO2 or maintain FiO2 while flow rate is change relative to the index change.
- FiO2 in the respiratory apparatus may be changed relative to respiratory rate change or relative to change in index.
- The mobile device is configured to determine a change in flow provided by the high flow respiratory apparatus required to improve the respiratory index, and presenting on the UI of the mobile device instructions to change flow rate.
- Flow rate is increased to improve the respiratory index.
- respiratory rateThe controller is configured to increase flow from a base flow rate if the trend (or change) in respiratory index indicates increased respiratory deterioration
- The controller is configured to reduce flow to a base flow rate if the respiratory index indicates reduced respiratory deterioration (or improvement).
- Optionally, any flow rate change is proportional to the change in the respiratory index
- Optionally, any flow rate change is a function of the change or function of the magnitude of change of the respiratory index.
- Display of respiratory therapy apparatus displays vector of respiratory index wherein vector indicates a change and trend over a period of time.
- If NHF is increased and respiratory rate drops without a decrease of FiO2 then this may indicate on the temporal effect of expiratory resistance only and device holds NHF rate or decrease it, for example by 5 L/min. If both FiO2 and respiratory rate are decreased the this an indication on the positive dynamic and NHF may start to decrease slowly from 60 L/min to 40-35 L/min to make the therapy more comfortable. Alternatively, in variable NHF the device starts to increase the expiratory pressure relief.

In an alternative, there is no separate assessment device, and the above embodiment is carried out completely on the respiratory support apparatus. The mobile device alternatively or additionally could be used as a remote control to control the respiratory support apparatus.

### 5.2 Alternative use cases

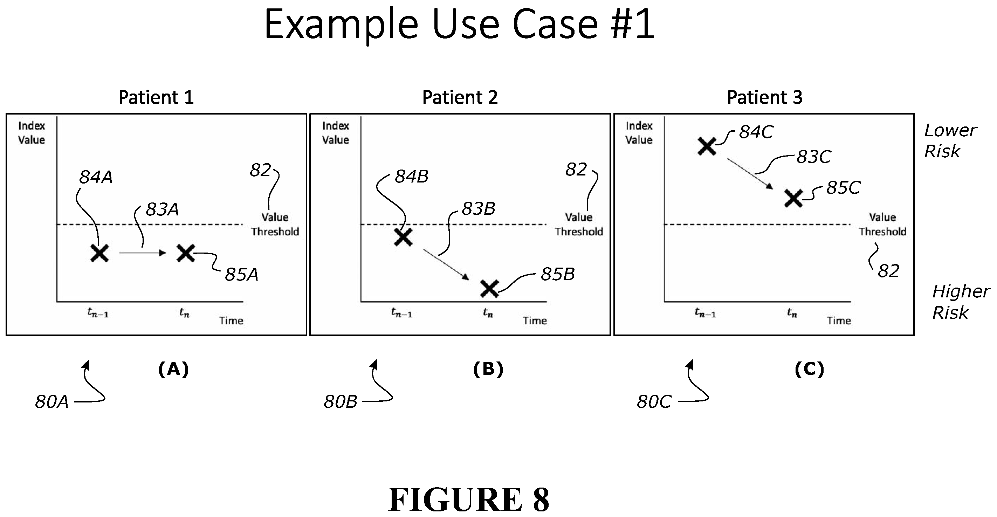

Figures 8A to 11E show alternative use cases that could be implemented on the apparatus of this section, or any other described or within the scope of this description.

Referring to Figures 8A to 8C, consider an example case in which the ROX values for three patients 80A to 80C are monitored over time. ROX is used by way of example, and the use cases could be generalised to any respiratory index.

The first patient's (patient 1) 80A current ROX value at tₙ 84A is on the high-risk side of a ROX value threshold 82 (that is below the threshold), but the ROX slope 83A (that is, the derivative of ROX over time tₙ-1 84A to tₙ 85A, or the slope between the current data ROX point tₙ 85A and the previous ROX data point 84A tₙ-1) is on the low-risk side of a slope threshold (the slope is zero in this case).

The second patient's (patient 2) 80B current ROX value 84B and ROX slope 83B are both on the high-risk side of their respective thresholds. That is, the ROX is below the ROX threshold 82, and the derivative of ROX over time tₙ-1 84B to tₙ 85B, or the slope between the current data ROX point tₙ 85B and the previous ROX data point 84B tₙ-1 is negative, so is trending worse).

The third patient's (patient 3) 80C current ROX value tₙ 84C is on the low-risk side of the value threshold 82 (below the threshold), but their ROX slope 83C is on the high-risk side of the slope threshold. That is, the derivative of ROX over time tₙ-1 84C to tₙ 85C, or the slope between the current data ROX point tₙ 85C and the previous ROX data point 84C tₙ-1 is negative, so is trending worse).

The ROX (values and slopes) for each of these patients 80A to 80C may indicate different states of wellbeing and may require different responses. For example, the first patient's ROX 85A may indicate that they are in a state that requires attention, but also that they have not recently deteriorated (a doctor may choose to prioritise other patients based on this information). The second patient's ROX indicates that their wellbeing is already in a high-risk state but may still be deteriorating - they need immediate attention from a doctor. The third patient's ROX 85C may indicate that their wellbeing is quickly deteriorating towards a high-risk state. This indication may allow a doctor to intervene early and prevent the patient's wellbeing from actually reaching a high-risk state (i.e. prevent their ROX value from decreasing to a high-risk value).

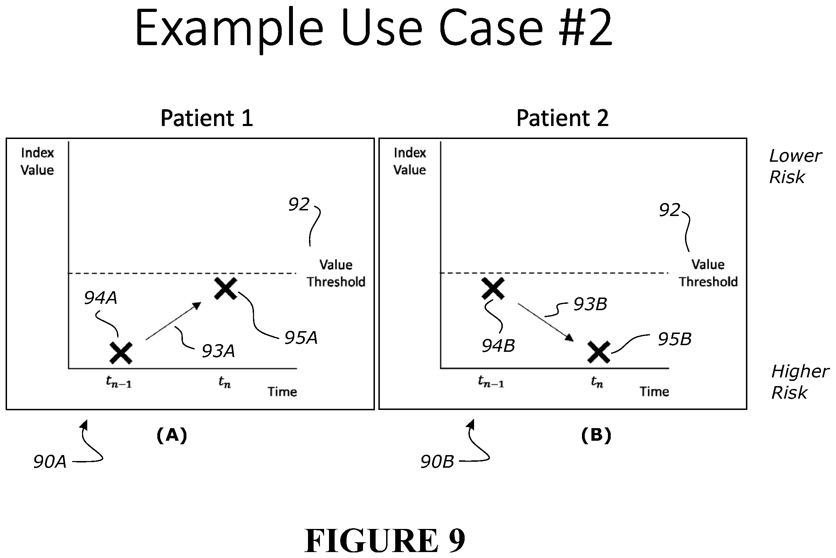

Referring to Figures 9A, 9B, consider another example case. Two patients (patient 1 and patient 2) have current ROX values tₙ-1 94A, 94B that are on the high-risk side of a ROX value threshold 92 (that is, below the threshold).

The first patient's (patient 1 90A) slope 93A indicates that their ROX value 94A is trending towards a lower-risk value 95A (towards the threshold). The second patient's (patient 2 90B) slope 93B indicates that their ROX value 94B is trending towards a higher-risk value 95B. Again, the ROX (values and slopes) for each of these patients indicate different states of wellbeing and may require different responses. The first patient 90A appears to be improving (trending better/positive 93A), so they may benefit from more therapy time at the same settings. The second patient 90B appears to be deteriorating further (trending worse/negative 93B), so they may need a change in therapy settings.

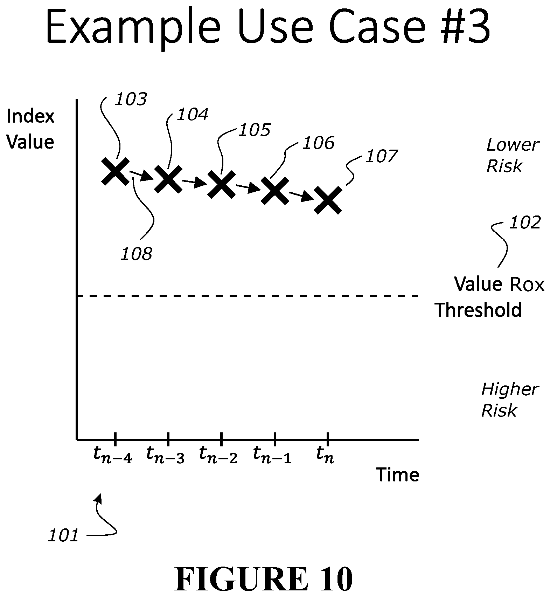

In addition to identifying short-term changes in patient wellbeing, the present system may use comparisons to thresholds to identify longer-term (i.e. slower) changes in patient wellbeing:
Referring to Figure 10, consider an example case in which a patient's 101 ROX value 103 to 107 decreases slowly over a number of days (tₙ-4 to tₙ). Although this decline may eventually result in the patient's ROX value dropping below a value threshold 102, the present system may be able to identify a decline in patient wellbeing before this occurs. By looking over multiple previous ROX-value data points 103 to 107, the present system may identify a (slow) decline in the ROX value - even if individual slopes e.g. 108 (between any two data points e.g. 103 to 104) are on the low-risk side of a slope threshold. The present system may then respond before the patient's ROX value actually drops below the value threshold - enabling an early (i.e. pre-emptive) intervention.

What this indicates is it is not just the slope/change over time that counts, but the relative magnitude of the ROX drop over a time period.

One exemplary embodiment is as follows. The present method and/or apparatus enable the determination of a respiratory index and/or respiratory status of a patient receiving high flow or other respiratory support. This provides information on a patient's condition when they are on respiratory therapy (e.g. high flow respiratory support such as nasal high flow). This provides information on whether the patient is stable, getting worse or getting better. This indirectly also can act as an indicator of effectiveness of the respiratory support. The information can be displayed numerically and/or graphically - e.g. as a vector. The information can be e.g.:
- the change over time of the respiratory index, akin to a first derivative with respect to time, and/or
- a trend over time of the change in time of the respiratory index - akin to second derivative with respect to time.

By reviewing the respiratory index and/or other parameters, and the change in respiratory index over time, assessment can be made about whether:
- a change in respiratory support is required, and/or
- what that change might be.

Some action can be taken based on the information, e.g.
- the change can be made,.
- instructions/alarm to change therapy, and/or
- apparatus automatically tries to change therapy.

Eventually if patient is not improving as based on the index (i.e. repeat of assessment phase), then alarms raised to escalate therapy

### 6. Clinician assessment and displayed information

As noted earlier, in many cases, it is the clinician that will assess the respiratory status based on information displayed on the assessment apparatus. By displaying various combinations of graphical and numerical representations of parameters and respiratory indexes, it is possible for a trained clinician to interpret these to gain an indication of respiratory status, and in particular the direction of respiratory state and what interventions might be required.

Various examples will now be discussed. These are not limiting, but rather provide indications of the source of information that might be displayed, and how it is used by clinician to assist respiratory status. The assessment by a clinician can include assessment criteria, which are criteria that allow the clinician to assess the respiratory index, patient parameters, change indicators and/or other assessment information to determine patient respiratory status. Assessment criteria can comprise and/or use relationship information, such as thresholds. These assessments can be used with any of the embodiments described herein.

To display assessment information, assessment criteria, relationship information and/or any other information, a user interface 54 can be used - on the assessment device (such as mobile device and/or therapy apparatus) and/or on a therapy apparatus. Numerical information, and graphical information can be displayed. Graphical information could take the form of graphs/plots in 2D or 3D. In 3D, optionally, one axis can be the time axis to show change of assessment information over time. User controls allow manipulation of the display. A touch screen might be used.

In a more general case, such as shown in Figure 2, the assessment method and apparatus comprise displaying, for the plurality of time points, the change in respiratory index over time for each of the plurality of time points. The clinician can then view this. A respiratory index threshold might be provided, to help assist determine whether the respiratory status is trending towards improvement or deterioration. Likewise, a change indicator, in this case a vector with a slope, can indicate the trend.

Referring to Figure 5, yet further information is provided. In this case vectors showing the change in respiratory index are plotted on a respiratory rate versus FiO2 graph. The ROX index threshold of 4.88 is also plotted on this graph. A positive slope (in this case) over time towards the top right hand of the figure indicates the patient is deteriorating. Alternatively, a negative slope over time towards the bottom left-hand corner indicates improvement (even though the ROX index starts in the "at risk" section, it is trending towards and passes the threshold into the improvement section).

In either case, the clinician can monitor change in respiratory index over time, with reference to a threshold, and in the case of Figure 5 also with the benefit of additional drill down information, where respiratory rate and FiO2 are also shown. This could be in addition to a process of calculating and comparing the various change information to relationship information (being thresholds, such as ROX threshold and/or change indicator thresholds such as a vector slope threshold). The information in Figures 2, 4 and 5 show a combination of graphical (e.g. plots and vectors etc.) in combination with numerical information.

In more general terms information is displayed on an interface, either on a respiratory apparatus, mobile device and/or other assessment apparatus, and can comprise: the respiratory index (e.g. ROX index) versus time, either graphically and/or numerically, one or more components of the respiratory index (.e.g. respiratory rate, SpO2, FiO2 or the like), alone, combined and/or options versus time, either graphically and/or numerically, and/or one or more vectors, slopes, angles , magnitudes, differences and/or other change indicators indicating change between two or more respiratory indexes and/or components thereof, over time or otherwise.

In doing so, a clinician can determines a patient respiratory status from a change in respiratory index over time by viewing: the respiratory index (e.g. ROX index) versus time, either graphically and/or numerically, one or more components of the respiratory index (.e.g. respiratory rate, SpO2, FiO2 or the like), alone, combined and/or options versus time, either graphically and/or numerically, and/or one or more vectors, slopes, angles, magnitudes, differences and/or other change indicators indicating change between two or more respiratory indexes and/or components thereof, over time or otherwise.

To help assess the information, the user might interact and/or manipulate the graphical interface to better use the information. This can comprise an apparatus and method for receiving input (e.g. user input) to revise the display and re-displaying information based on the user input, comprising one or more of: receiving input to display one or more components of the respiratory index, and displaying the one or more components of the respiratory index (.e.g. respiratory rate, SpO2, FiO2 or the like), alone, combined and/or options versus time, either graphically and/or numerically, and/or receiving input to display, zoom and/or move the display, and displaying, or redisplaying a zoomed and/or moved version of: respiratory index (e.g. ROX index) versus time, either graphically and/or numerically. one or more components of the respiratory index, alone, combined and/or options versus time, either graphically and/or numerically, and/or one or more vectors, slopes, angles , magnitudes, differences and/or other change indicators indicating change between two or more respiratory indexes and/or components thereof, over time or otherwise.

As an example, the patient might be able to click on the information in Figure 2 to receive the information of Figure 5. They could then zoom in and/or move Figure 5 to better view the information.

A clinician determines a patient respiratory status from a change in respiratory index over time by any one or a combination of the following:
comparing one or more respiratory index(es) and/or a change in respiratory index, relative to one or more threshold(s) - see e.g. Figure 2, 4, 5
comparing one or more change indicators relative to one or more threshold(s),
comparing one or more respiratory index(es) and/or a change in respiratory index, relative to one or more other respiratory index(es) and/or a change in respiratory index and/or relative to one or more other one or more change indicators.
comparing one or more change indicators relative to one or more other change indicators and/or one or more respiratory index(es) and/or a change in respiratory index.
considering one or more:
   respiratory indexes,
   change in respiratory indexes over time,
   change in, change in respiratory indexes over time, and/or
   change indicators.

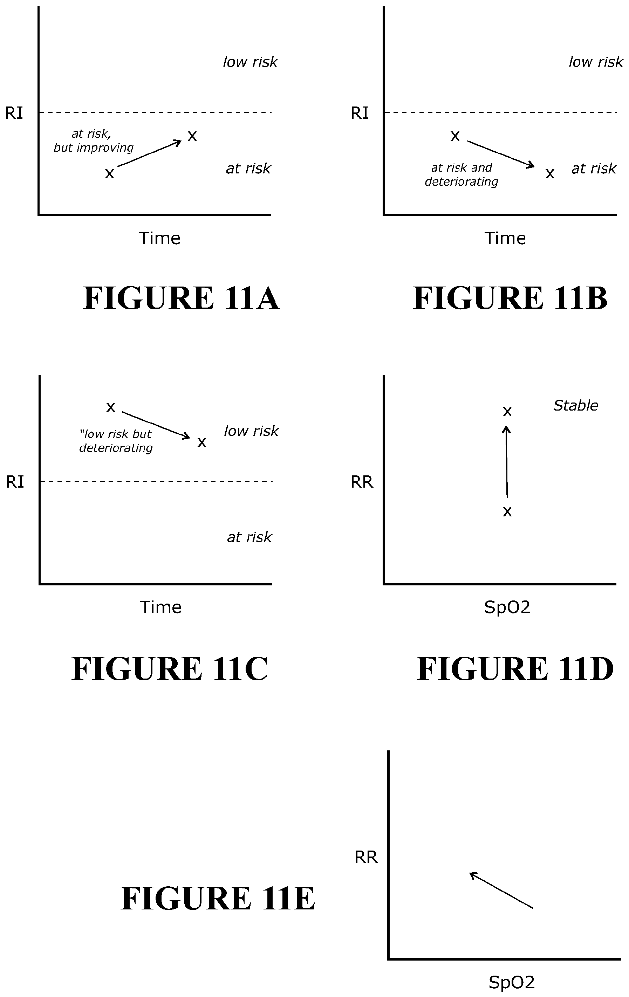

Particular nonlimiting examples are shown in Figures 11A to 11E

A clinician determines a patient respiratory status as "at risk but improving" if: ROX index is below a threshold but the ROX index change indicator shows a trend towards lower risk. See Figure 11A. If determined as such, an assessment apparatus might also provide an indication, such as an initial alarm and display message that indicates the patient is at risk but improving.

A clinician determines a patient respiratory status as "at risk and deteriorating" if: ROX index is below a threshold and the ROX index change indicator is showing a trend toward higher risk. See Figure 11B. If determined as such, then an assessment apparatus provides an indication, such as an alarm and display message that indicates the patient is at risk and deteriorating.

A clinician determines a patient respiratory status as "not at risk (or low risk) but deteriorating" if: ROX index is above a threshold but the ROX index change indicator is showing a trend toward higher risk. See Figure 11C. If determined as such, then an assessment apparatus provides an indication, such as a quiet alarm, and then alarms loudly if/when the ROX index drops below the threshold.

A clinician determines a patient respiratory status is not changing significantly, but still assesses a potential issue if: respiratory rate is trending upwards (by more than threshold slope or other change indicator) even if SpO2 is stable, see Figure 11D. If determined, then a suitable message is displayed on screen.

A clinician determines a patient respiratory status as "deteriorating" if:
respiratory rate is trending upwards (by more than a threshold slope or other change indicator) and SpO2 is trending downwards. See Figure 11E. If determined, a suitable alarm is activated.

A clinician determines a patient respiratory status from ROX index compared to one or more thresholds. E.g. see Figure 2.

A clinician determines a patient respiratory status from:
respiratory rate,
SpO2, and/or
FiO2
based on one or more thresholds.

A clinician determines a patient respiratory status from a change over time of:
respiratory index, and/or patient parameters, such as respiratory rate, SpO2, and/or FiO2. See Figure 5.

A clinician determines a patient respiratory status from a change indicator such as slope, magnitude, and/or angle between respiratory index values at a plurality of time points. See Figure 5.

A clinician determines a patient respiratory status from a change indicator such as slope, magnitude, and/or angle between patient parameters, such as respiratory rate, SpO2, and/or FiO2, at a plurality of time points.

A clinician determines a patient respiratory status from the length of time a respiratory index and/or change indicator takes to change and/or the magnitude of the change over a threshold time.

A clinician determines a patient respiratory status from the time taken to for a respiratory index and/or change indicator by a threshold amount.

The assessment information will be based on information obtained at a plurality of time points. These may be continuous time points. They might be non-continuous time points (discrete). Likewise, the display of assessment information may be made at continuous points, in time and/or on the display. Or, the display of information might be made non-continuously, in time and/or on the display. Where the information or display are non-continuous in time, the time points might be separated by less than a second, 1 second, seconds, less than a minute, 1 minute or minutes, or any point between 1 to 59 minutes, less than an hour, one hour, hours, or any point between 1 to 24 hours, less than a day, one day, or days. Thee time points could be regular or irregular.

If the respiratory index has improved by a certain threshold then the flow rate could be dropped to a base flow rate or flow rate reduced based on the rate of change of the respiratory index. For example, a change in flow may be proportional or may be defined by a function that relates flow rate change to the change in the respiratory index. The function may be a decay function or log function or a hyperbolic function. When a clinician makes a respiratory status/index assessment, they can also determine a suitable respiratory support change, if required.

If the apparatus makes a respiratory status/index assessment, it might alert the clinician via messages, alerts, alarms, information, or other indicators as described herein. This alerts the clinician to the requirement of a respiratory support change, but may not actually suggest what the change should be. They could manually make the change by operating a respiratory support apparatus. Alternatively, or additionally, the assessment apparatus might suggest what the respiratory support change should be. This might be through any sort of indicators, such as alerts, alarms, messages information or the like. Again, the clinician could make the change. It further, the respiratory support apparatus might automatically make the change required.

Optionally, the respiratory apparatus may comprise a communication interface that is configured to transmit information to a mobile device (e.g. smartphone or tablet) associated with a clinician or healthcare professional and/or transmit information to a remote patient monitoring system. The remote patient monitoring system may comprise one or more servers, client devises, memory units, databases and/or other components that allow management of patient information, generation of reports of patient's health status and allow alerts to be sent to and/or accessed by the patient and/or clinician. The change in respiratory index may be transmitted to the mobile device and/or to the remote patient monitoring system.

The respiratory index measurements and change in respiratory index may be incorporated into a patient report that includes measured patient parameters e.g. SpO2, flow rate, humidity set point and usage hours and the change in respiratory index and respiratory index measured values over time.

The change in respiratory index allows a clinician to assess if the current therapy being provided is being effective and also allows a clinician to make a change in the therapy provided. In one example the operational parameters of the respiratory support apparatus (e.g. prescription settings) may be remotely updated based on the change in the respiratory index.

### 7. Advantages

The one or more of the embodiments above can provide one or more of the following advantages:
- Visual tracking of a respiratory trend to enable a clinician to make decisions about respiratory support. E.g. embodiments can show a trend that is a negative trend and/or a negative trend that is greater than threshold.
- Provision of an alarm that a patient is trending in the wrong direction so decisions can be made about respiratory support.
- Device-led decisions based on respiratory trends that suggest a patient is likely to have an adverse health effect and requires a particular level of respiratory support or a particular type of respiratory support, including being escalated to a more invasive respiratory support. The device controller may automatically change the level of respiratory support or generate an alarm.
- Enabling decisions about escalation of respiratory support to be made soon enough so that the escalation will provide a good outcome, but not so soon that the escalation might be provided unnecessarily early.
- An automated method of determining patient respiratory status and change in respiratory state based on the change or rate of change of respiratory index. This provides an early warning as compared to more time consuming and/or invasive diagnostic methods.
- An automated, minimally invasive method of determining a respiratory index and assessing respiratory status.
- A respiratory support apparatus that acts as an integrated sensing unit that can be used for the assessment phase in hospital or home.
- Improved decision making.
- Faster escalation of respiratory support if respiratory status and/or respiratory index worsens.
- Remote monitoring of conditions (e.g. monitoring homecare patients) to help with managing therapy. The embodiments can help assess patients that are remote to clinicians based on the change in the respiratory index.
- The respiratory apparatus functions as a non-invasive sensing block/device to collate devices. Respiratory index is calculated in the device. Respiratory index changes can be calculated in the respiratory support apparatus too or in a remote patient monitoring device.
- Assessing effectiveness of NHF therapy and also helping in determining if a patient in a remote setting may suffer from respiratory failure and provide early warning of this.
- Knowing where to set a respiratory index threshold value (to distinguish between a prediction of NHF success and a prediction of NHF failure under the current therapy conditions) requires empirical data for different patient groups. This is because a threshold value that effectively distinguishes between predicted success or failure for patients with COPD may not effectively distinguish this for patients with pneumonia, for example. Using the change in respiratory index value to assess patients overcomes this issue - as no threshold respiratory index value is needed.
- A clinician could miss a potentially problematic change in a patient's condition if they only monitor the value of the patient's respiratory index (and not real-time changes in this value). For example, a potentially problematic increase in a patient's respiratory rate may not result in a change in their respiratory index value if FiO2 decreases at the same time. Displaying a plot of the patient's respiratory rate, against their SpO2 divided by their FiO2, would allow a clinician to see that the patient's respiratory rate is increasing, even if their index value is not. Likewise, an alarm that was based on the slope, of one or more vectors joining data points on such a plot, could activate if such a change in respiratory rate occurred.

This divisional application is divided from EP21838958.3 (the 'parent application') and the divisional specification as filed comprises the content of the parent application, including the original claims recited as 'representative features' below. The scope of this disclosure therefore includes the full content of the parent application. In any case, protection may be sought for any features disclosed in the parent application as filed.

### REPRESENTATIVE FEATURES

1. A method of assessing a patient receiving respiratory support during a session to determine a respiratory status comprising:
   receiving from one or more sensors, for a plurality of time points, one or more patient parameters for a patient, comprising at least one respiratory parameter,
   determining in a controller:
      for each time point, a respiratory index from the one or more patient parameters, and
      a change in respiratory index over time, and
      determining, from the change in respiratory index over time, a patient respiratory status.
2. A method according to clause 1 wherein the patient is receiving respiratory support, and optionally the respiratory support is:
   high flow respiratory support,
3. A method according to clause 1 or 2 wherein the session:
   is a treatment session,
   a day or part thereof,
   a night or part thereof,
   sub-sessions,
   a length of time.
4. A method according to any preceding clause wherein the one or more patient parameters are one or more lung mechanics parameters and one or more oxygenation parameters.
5. A method according to clause 4 wherein a lung mechanics parameter can be one or more of:
   - Respiratory rate
   - Expiratory time
   - Minute ventilation
6. A method according to clause 4 or 5 wherein a oxygenation parameter can be one or more of:
   - FiO2
   - FdO2
   - O2 fraction
   - SpO2
7. A method according to any preceding clause wherein the respiratory index is ROX index.
8. A method according to clause 7 wherein components of the ROX index are:
   respiratory rate,
   SpO2, and/or
   FiO2, FdO2, and/or O2 fraction.
9. A method according to clause 5 or 8 wherein a respiratory rate is determined by the controller from one or more patient parameters received from the one or more sensors.
10. A method according to any preceding clause further comprising indicating and/or making a change in respiratory support based on the respiratory status and/or respiratory index.
11. A method according to any preceding clause comprising displaying ROX index numerically and/or graphically.
12. A method according to any preceding clause wherein determining a change in respiratory index over time comprises:
   for a plurality of time points, determining a change in respiratory index over time for each of the plurality of time points.
13. A method according to clause 11 or 12 further comprising displaying, for the plurality of time points, the change in respiratory index over time for each of the plurality of time points.
14. A method according to clause 11, 12 or 13 wherein determining a patient respiratory status from the change in respiratory index over time comprises monitoring a change, over the plurality of points in time, of the change in respiratory index over time.
15. A method according to clause 14 wherein monitoring a change, over the plurality of time points, of the change in respiratory index over time comprises, for the plurality of time points:
   viewing the displayed change, over the plurality of points in time, of the change in respiratory index over time ,and/or
   calculating and comparing the change to relationship information
16. A method according to any one of clauses 11 to 15 further comprising displaying a respiratory index threshold and/or change indicator threshold.
17. A method according to any preceding clause wherein determining a change in respiratory index over time comprises determining a trend in the respiratory index.
18. A method according to clause 17 wherein the trend comprises a plurality of instantaneous trends, and determining a trend comprises determining a plurality of instantaneous trends over time.
19. A method according to clause 17 or 18 wherein a trend or an instantaneous trend is represented with a trend parameter comprising magnitude and a direction, and optionally could be in the form of:
   a vector, or
   a slope and magnitude.
20. A method according to any preceding clause further comprising communicating the determined change in respiratory support to:
   a clinician, for example in the form of a message, alarm, respiratory status, respiratory index, and/or
   a respiratory support apparatus.
21. A method according to any previous clause wherein the one or more sensors comprise:
   one or more sensors arranged to sense a flow path of a respiratory support apparatus, and/or
   one or more sensors arranged to sense parameters of a patient,
      and
   the controller receives the one or more patient parameters from the one or more sensors.
22. A method according to any preceding clause further comprising displaying on an interface, either on a respiratory apparatus, mobile device and/or other assessment apparatus one or more of:
   the respiratory index versus time, either graphically and/or numerically,
   one or more components of the respiratory index (e.g. respiratory rate, SpO2, FiO2 or the like), alone, combined and/or options versus time, either graphically and/or numerically, and/or
   one or more vectors, slopes, angles, magnitudes, differences and/or other change indicators indicating change between two or more respiratory indexes and/or components thereof, over time or otherwise.
23. A method according to clause 22 further comprising receiving input (e.g. user input) to revise the display and re-displaying information based on the user input, comprising one or more of:
   receiving input to display one or more components of the respiratory index, and displaying the one or more components of the respiratory index alone, combined and/or options versus time, either graphically and/or numerically, and/or
   receiving input to display, zoom and/or move the display, and displaying, or redisplaying a zoomed and/or moved version of:
      respiratory index (e.g. ROX index) versus time, either graphically and/or numerically,
      one or more components of the respiratory index, alone, combined and/or options versus time, either graphically and/or numerically, and/or
      one or more vectors, slopes, angles, magnitudes, differences and/or other change indicators indicating change between two or more respiratory indexes and/or components thereof, over time or otherwise.
24. A method according to clause 22 or 23 wherein a clinician determines a patient respiratory status from a change in respiratory index over time by viewing
   the respiratory index (e.g. ROX index) versus time, either graphically and/or numerically,
   one or more components of the respiratory index (e.g. respiratory rate, SpO2, FiO2 or the like), alone, combined and/or options versus time, either graphically and/or numerically, and/or
   one or more vectors, slopes, angles, magnitudes, differences and/or other change indicators indicating change between two or more respiratory indexes and/or components thereof, over time or otherwise.
25. A method according to any preceding clause wherein a clinician determines a patient respiratory status from a change in respiratory index over time by any one or a combination of the following:
   comparing one or more respiratory index(es) and/or a change in respiratory index, relative to one or more threshold(s),
   comparing one or more change indicators relative to one or more threshold(s),
   comparing one or more respiratory index(es) and/or a change in respiratory index, relative to one or more other respiratory index(es) and/or a change in respiratory index and/or relative to one or more other one or more change indicators,
   comparing one or more change indicators relative to one or more other change indicators and/or one or more respiratory index(es) and/or a change in respiratory index,
   considering one or more:
      respiratory indexes,
      change in respiratory indexes over time,
      change in, change in respiratory indexes over time, and/or
      change indicators.
26. A method according to any preceding clause wherein upon determining a patient respiratory status, one or more of the following can occur to indicate respiratory status:
   an alarm is sounded and/or a message is displayed, which indicate the respiratory status, alert the clinician and/or indicate action required, and/or
   a change in therapy is actioned, automatically and/or manually.
27. An apparatus for assessing a patient receiving respiratory support during a session to determine a respiratory status comprising:
   one or more sensors, or inputs for one or more sensors for receiving from for a plurality of time points, one or more patient parameters for a patient, comprising at least one respiratory parameter,
   a controller for determining:
      for each time point, a respiratory index from the one or more patient parameters, and
      determining, from the change in respiratory index over time, a patient respiratory status, and/or
      displaying on a display a change in respiratory index over time for a user to determine a patient respiratory status.
28. An apparatus according to clause 27 wherein the patient is receiving respiratory support, and optionally the respiratory support is:
   high flow respiratory support.
29. An apparatus according to clause 27 or 28 wherein:
   the assessment apparatus provides the respiratory support, or
   the assessment apparatus is separate to a respiratory support apparatus.
30. An apparatus according to clause 27, 28 or 29 wherein the session:
   is a treatment session,
   a day or part thereof,
   a night or part thereof,
   sub-sessions,
   a length of time.
31. An apparatus according to any one of clauses 27 to 30 wherein the one or more patient parameters are one or more lung mechanics parameters and one or more oxygenation parameters.
32. An apparatus according to clause 31 wherein a lung mechanics parameter can be one or more of:
   - Respiratory rate
   - Expiratory time
   - Minute ventilation
33. An apparatus according to clause 31 or 32 wherein a oxygenation parameter can be one or more of:
   - FiO2
   - FdO2
   - O2 fraction
   - SpO2
34. An apparatus according any one of clauses 27 to 33 wherein the respiratory index is ROX index.
35. An apparatus according to clause 34 wherein components of the ROX index are:
   respiratory rate,
   SpO2, and/or
   FiO2, FdO2, and/or O2 fraction.
36. An apparatus according to clause 35 wherein a respiratory rate is determined by the controller from one or more patient parameters received from the one or more sensors.
37. An apparatus according any one of clauses 27 to 36 wherein the respiratory index is ROX index, determined from respiratory rate, FiO2 and/or SpO2 .
38. An apparatus according any one of clauses 27 to 37 comprising displaying ROX index numerically and/or graphically on the display.
39. A apparatus according any one of clauses 27 to 38 wherein determining a change in respiratory index over time comprises:
   for a plurality of time points, determining a change in respiratory index over time for each of the plurality of time points.
40. A apparatus according to clause 39 further comprising displaying, for the plurality of time points, the change in respiratory index over time for each of the plurality of time points.
41. An apparatus according to clause 40 wherein determining from the change in respiratory index over time, comprises the controller calculating and comparing the change to relationship information.
42. An apparatus according to any one of clauses 27 to 41 further comprising the controller displaying:
   a respiratory index threshold and/or change indicator threshold.
43. An apparatus according any one of clauses 27 to 42 further comprising communicating the determined change in respiratory support to:
   a clinician, for example in the form of a message, alarm, respiratory status, respiratory index, and/or
   a respiratory support apparatus.
44. An apparatus according any one of clauses 27 to 43 wherein the one or more sensors comprise:
   one or more sensors arranged to sense a flow path of a respiratory support apparatus, and/or
   one or more sensors arranged to sense parameters of a patient,
      and
   the controller receives the one or more patient parameters from the one or more sensors.
45. An apparatus according any one of clauses 27 to 44 wherein the apparatus is one or more of a:
   respiratory apparatus,
   mobile device,
   a remote monitoring system,
   either alone or integrated.
46. An apparatus according to any one of clauses 27 to 45 comprising the sensors.
47. A system for assessing a patient receiving respiratory support during a session to determine a respiratory status comprising:
   An apparatus according to any one of clauses 27 to 46 carrying out a method according to any one of clauses 1 to 26.

## Claims

1. A respiratory apparatus for nasal high flow therapy, the respiratory apparatus comprising:
a flow generator and a humidifier configured to deliver a humidified high flow of gas to a patient through a nasal patient interface;
one or more sensors, or one or more inputs for the one or more sensors, configured to obtain, for each of a plurality of time points, a fraction of inspired oxygen value, an oxygen saturation value, and a respiratory rate of the patient;
a controller configured to automatically calculate, for each of the plurality of time points, a ROX index from the fraction of inspired oxygen value, the oxygen saturation value, and the respiratory rate; and
a user interface configured to automatically display a plot of the ROX index over time.

2. The respiratory apparatus according to claim 1, wherein the controller is configured to determine a respiratory status of the patient based on the ROX index over time.

3. The respiratory apparatus according to claim 2, wherein the controller is configured to determine the respiratory status by comparing at least one of the ROX index or a change of the ROX index over time with relationship information.

4. The respiratory apparatus according to claim 3, wherein the relationship information comprises at least one of a ROX index threshold or a change indicator threshold.

5. The respiratory apparatus according to any one of claims 2 to 4, wherein the controller is configured to assess an efficacy of the nasal high flow therapy based on the respiratory status.

6. The respiratory apparatus according to claim 5, wherein the controller is configured to determine that the nasal high flow therapy is not effective when the respiratory status indicates that the patient is deteriorating.

7. The respiratory apparatus according to claim 6, wherein the controller is configured to generate an alarm when the nasal high flow therapy is determined to be not effective.

8. The respiratory apparatus according to any one of claims 2 to 4, wherein the controller is configured to identify that the patient requires escalation of respiratory support based on the respiratory status.

9. The respiratory apparatus according to claim 8, wherein the controller is configured to generate an alarm or a message indicating that escalation of respiratory support is required.

10. The respiratory apparatus according to any one of claims 2 to 4, wherein the controller is configured to identify that the patient is not responding to the nasal high flow therapy based on repeated assessments of the respiratory status.

11. The respiratory apparatus according to claim 10, wherein the controller is configured to generate an alarm or a message indicating that the patient is not responding to the nasal high flow therapy.

12. The respiratory apparatus according to any one of claims 7, 9, or 11, wherein the alarm or the message is configured to prompt a clinician to change the nasal high flow therapy.

13. The respiratory apparatus according to claim 12, wherein the change comprises escalation of respiratory support.

14. The respiratory apparatus according to claim 8 or 13, wherein the escalation of respiratory support comprises increasing a flow rate or an oxygen concentration delivered by the respiratory apparatus.

15. The respiratory apparatus according to claim 8 or 13, wherein the escalation of respiratory support comprises transferring the patient to non-invasive pressure respiratory support or invasive respiratory support.
